# EUROPEAN PATENT SPECIFICATION

(11) **EP 4 106 614 B1**
(45) Date of publication and mention of the grant of the patent: **26.06.2024**
(21) Application number: 21706903.8
(22) Date of filing: 18.02.2021
(51) Int. Cl.: A61B 5/00, G06F 3/0482, G06F 3/0488, H04N 1/00, A61B 1/00, G16H 30/20, G16H 40/63

(54) **CAPTURING AND BROWSING IMAGES IN A MEDICAL VISUALISATION SYSTEM**
AUFNAHME UND BROWSEN VON BILDERN IN EINEM MEDIZINISCHEN VISUALISIERUNGSSYSTEM
CAPTURE ET NAVIGATION D'IMAGES DANS UN SYSTÈME DE VISUALISATION MÉDICALE

(30) Priority: 21.02.2020 DK PA202070111
(43) Date of publication of application: 28.12.2022
(73) Proprietor: Ambu A/S, 2750 Ballerup (DK)
(72) Inventor: UBBESEN, Line, Sandahl, 2840 Holte (DK)
(74) Representative: COPA Copenhagen Patents
(86) International application number: PCT/EP2021/053963
(87) International publication number: WO 2021/165360

(56) References cited:
- WO-A2-2012/033936
- US-A1- 2006 152 516
- US-A1- 2019 033 571
- US-A1- 2019 238 791

## Description

The present disclosure relates to a visualisation device, such as an endoscope and a medical visualisation system, such as an endoscope system, comprising a visualisation device. More specifically the present disclosure relates to a graphical user interface and a monitor device having such graphical user interface for interacting with the medical visualisation system.

### BACKGROUND

A visualisation device may be utilized to visually examine certain areas of the body of a person, such as inside a body cavity of the person. For example, a visualisation device may be used to inspect the airways, the digestive tract, or the intestines.

A visualisation device may be provided with a camera and be attached to a monitor device, such as a monitor with a display screen, a video output from the camera of the visualisation device may be received and displayed at the monitor device, thereby allowing an operator to control the visualisation device to inspect an area of interest. Such a visualisation device is disclosed e.g. in WO 2012/033936A.

For example, a visualisation device may be an endoscope, such as a disposable endoscope. An endoscope comprises an operating handle at the proximal end and an insertion tube extending from the handle towards the distal end. The handle is configured to be held by an operator and inter alia comprises externally protruding operating members connected to internal control means allowing the operator to control the movement of a bending section at the distal end of the insertion tube, while advancing the distal end of the insertion tube to a desired location e.g. within a body cavity of a person. By means of an attached monitor device, such as a monitor with a display screen, the location to which the distal end has been advanced may be inspected using the endoscope.

The monitor device of a medical visualisation system may be provided with some functionality, such as ability to save still images and/or video sequences of the view from the attached visualisation device. Furthermore, the monitor device may comprise some image processing capabilities, and may be configured to output a video or image output, e.g. to an external display.

### SUMMARY

The present disclosure relates to a visualisation device, such as an endoscope, and a visualisation system, such as an endoscope system. Particularly, but not exclusively the visualisation device may be a disposable camera endoscope. Alternatively, the visualisation device may be a video laryngoscope, an endotracheal tube and/or a laryngeal mask. The visualisation system may further comprise a monitor device for being connected to the visualisation device, e.g. the monitor device may be configured to receive image data from the visualisation device. The present disclosure further relates to a graphical user interface for such monitor device of a medical visualisation system.

It is an object of the present disclosure to provide a solution which at least improve the solutions of the prior art. Particularly, it is an object of the present disclosure to provide a graphical user interface for a medical visualisation system which facilitates and enhances human interaction with the system.

It is a further object of the present disclosure to provide a system and method facilitating enhanced control and usability of a medical visualisation system.

Accordingly, a medical visualisation system of the present invention is defined in claim 1.

The medical visualisation system comprises a visualisation device, such as an endoscope, such as a disposable endoscope. Alternatively, the visualisation device may be a video laryngoscope, an endotracheal tube and/or a laryngeal mask. The visualisation device has an image sensor configured to generate image data indicative of a view from the visualisation device. The medical visualisation system may comprise a plurality of visualisation devices each having an image sensor configured to generate image data indicative of a view from the visualisation device. The plurality of visualisation devices may include a first visualisation device and/or a second visualisation device. The first visualisation device may comprise a first image sensor configured to generate image data indicative of a view from the first visualisation device. The second visualisation device may comprise a second image sensor configured to generate image data indicative of a view from the second visualisation device. The image sensor(s) may be any sensor capable of detecting and conveying information used to make an image. For example, the image sensor(s) may comprise a CCD or CMOS sensor, or similar. The image sensor(s) may generate image data corresponding to a square image, i.e. having equal height and width. For example, the image data generated by the image sensors may correspond to a 300x300 pixel image, or a 400x400 pixel image, or a 600x600 pixel image, or a 800x800 pixel image. Alternatively or additionally, the image sensor may generate image data corresponding to a non-square image, which is cropped to form a square image, such as a square image having 300x300 pixels, 400x400 pixels, 600x600 pixels, or 800x800 pixels.

The medical visualisation system further comprises a monitor device receiving and/or being operable to receive the image data generated by the image sensor. The monitor device may receive and/or be operable to receive the image data, e.g. as the image data is being generated, e.g. within limitation of the hardware. The monitor device comprises a first housing extending in a first direction from a first housing side to a second housing side and in a second direction perpendicular to the first direction from a third housing side to a fourth housing side. The monitor device comprises a display, e.g. a touch sensitive display. The display may be accommodated in the first housing. The display may have a first length in the first direction and a second length in the second direction. The second length may be longer than the first length, e.g. the display may be a 16:9 or 16:10 display. Alternatively, the first length may be longer than the second length, or the first length and the second length may be substantially the same. The touch sensitive display may be any suitable type of touch display, e.g. capacitive touch display or resistive touch display.

The monitor device may comprise one or more connection ports configured to receive a connector of the visualisation device. The connection ports and the corresponding connector of the visualisation device may be a proprietary plug and socket connectors, or any standard connector capable of transmitting therethrough at least the image data from the image sensor. Furthermore, the connector and connection ports may be configured to supply power to the components of the visualisation device.

The one or more connection ports may be arranged on the first housing. The one or more connection ports may be provided on the third housing side, and/or on the fourth housing side. The monitor device may comprise an on/off button. The on/off button may be arranged on the first housing. The on/off button may be provided on the third housing side or on the fourth housing side. The one or more connection ports may be provided on the third housing side and the on/off button may be provided on the fourth housing side.

The monitor device may establish connection to a visualisation device, such as the first visualisation device and/or the second visualisation device. Establishing connection to the visualisation device may include receiving a device connector of the respective visualisation device in a connection port of the one or more connection ports of the monitor device. Establishing connection to a visualisation device may include obtaining device identifier information from a device identifier (e.g. EPROM, QR-code, NFC, RFID or similar) of the visualisation device. For example, establishing connection to the first visualisation device may include obtaining first device identifier information from a first device identifier of the first visualisation device and/or establishing connection to the second visualisation device may include obtaining second device identifier information from a second device identifier of the second visualisation device.

The monitor device may comprise a processing unit and memory. The processing unit and/or the memory may be accommodated in the first housing. Alternatively, the monitor device may comprise a second housing, and the processing unit and/or the memory may be accommodated in the second housing. The monitor device may comprise an orientation sensor, e.g. for determining the orientation of the monitor device, such as of the first housing, relative to gravity. The orientation sensor may comprise one or more accelerometers and/or a gyroscope. The orientation sensor may be accommodated in the first housing. The processing unit may be connected to the touch sensitive display to control display of information with the touch sensitive display, and the processing unit may be adapted to receive a signal from the touch sensitive display indicative of touch inputs on the touch sensitive display. Thereby, the monitor device may detect user inputs, e.g. in the form of touch inputs, with the touch sensitive display. Touch inputs may, for example, comprise single tap(s), double tap(s), or swipe(s) on the touch sensitive display. The processing unit may be connected to the orientation sensor to receive an orientation signal indicative of the orientation of the monitor device, such as of the first housing of the monitor device. The processing unit may be connected to the memory and be adapted to read and write data from and to the memory.

The monitor device may comprise a power unit for powering the monitor device. The power unit may comprise a rechargeable battery and/or a power connection for connecting the power unit to an external power supply, such as a conventional AC power socket. The power unit may be accommodated in the first housing. Alternatively, the power unit may be accommodated in the second housing.

The monitor device comprises a graphical user interface. The monitor device and/or the processing unit of the monitor device displays with the touch sensitive display the graphical user interface. The graphical user interface comprises one or more portions, such as a plurality of portions. The portions may be non-overlapping portions, such as a plurality of non-overlapping portions. The portions may include a first portion and/or a second portion. The portions may further include a third portion and/or a fourth portion. The second portion and/or the fourth portion may be designated as background portions, e.g. the second portion may be a first background portion and/or the fourth portion may be a second background portion. Each of the plurality of portions may extend substantially throughout the first length in the first direction. The first portion may be arranged between the fourth portion and the second portion along the second direction. The fourth portion may be arranged between the third portion and the first portion along the second direction. The third portion may be arranged between a side of the first housing, e.g. the third housing side, and the fourth portion along the second direction. The second portion may be arranged between another side of the first housing, e.g. the fourth housing side, and the first portion along the second direction. The first portion and the fourth portion may be arranged between the second portion and the third portion along the second direction. The first portion of the graphical user interface may be square. The first portion of the graphical user interface may occupy the centre of the touch sensitive display. The first portion of the graphical user interface may be larger along the second direction than the second portion, the third portion and/or the fourth portion, individually and/or collectively. The first portion of the graphical user interface may extend throughout more than 40% of the second length in the second direction, such as more than 50% of the second length in the second direction, such as more than 60% of the second length in the second direction.

The monitor device displays a live representation of the image data, e.g. within the first portion of the graphical user interface. The live representation of the image data may be displayed, e.g. by the processing unit, with the touch sensitive display, e.g. within the first portion of the graphical user interface.

The visualisation device, e.g. an endoscope, may comprise a handle and an elongated flexible member extending from the handle to a distal end. The image sensor may be arranged at the distal end of the elongated flexible member. The image data may be indicative of a view from the distal end of the elongated flexible member. The handle may comprise a control button adapted to receive an input in a first input direction and/or in a second input direction. The first input direction and the second input direction may be opposite. The touch input in the first input direction may cause a distal portion of the elongated flexible member to bend in a first bending direction and/or may cause movement of the image sensor in a first image sensor direction. The touch input in the second input direction may cause the distal portion of the elongated flexible member to bend in a second bending direction and/or may cause movement of the image sensor in a second image sensor direction. The live representation of the image data may have directions corresponding to directions of the image sensor generating the image data. The first bending direction may correspond to a first image direction of a representation of the image data, such as the live representation of the image data. The second bending direction may correspond to a second image direction of the representation of the image data, such as the live representation of the image data. The first image direction and/or the second image direction may be parallel to the first direction of the first housing.

The monitor device provides one or more actionable items. One or more actionable items may be displayed, e.g. by the processing unit, with the touch sensitive display, e.g. within the second portion of the graphical user interface. One or more actionable menu items may be displayed, e.g. by the processing unit, with the touch sensitive display, e.g. within the third portion of the graphical user interface. A battery indicator may be displayed, e.g. by the processing unit, with the touch sensitive display, e.g. within the third portion of the graphical user interface. A time indicator may be displayed, e.g. by the processing unit, with the touch sensitive display, e.g. within the third portion of the graphical user interface.

The one or more actionable items, e.g. displayed within the second portion of the graphical user interface of the monitor device, may comprise an image capture button and/or a video capture button. In response to activation of the image capture button, e.g. by a user providing a touch input, e.g. a single tap, at the respective location of the touch sensitive display, an image data file corresponding to the image data received when the image capture button was activated may be stored, e.g. in memory of the monitor device. In response to activation of the video capture button, e.g. by a user providing a touch input, e.g. a single tap, at the respective location of the touch sensitive display, a video sequence of image data corresponding to the image data received when the video capture button was activated may be stored, e.g. in memory of the monitor device. A first activation of the video capture button may start collection of image data for the video sequence, and a second activation of the video capture button (subsequent to the first activation of the video capture button) may stop the collection of image data for the video sequence. The stored video sequence may correspond to the image data received between the first activation and the second activation of the video capture button. The video capture button may be displayed in a first appearance prior to the first activation and after the second activation. The video capture button may be displayed in a second appearance after the first activation and before the second activation.

The monitor device may, e.g. in response to establishing connection to a visualisation device, such as the first visualisation device, open a procedure session. Establishing connection to a visualisation device may include obtaining device identifier information from a device identifier (e.g. EPROM, QR-code, NFC, RFID or similar) of the visualisation device. In response to establishing connection to the first visualisation device the monitor device may open a first procedure session corresponding to the first device identifier information obtained from the first device identifier of the first visualisation device. In response to establishing connection to the second visualisation device the monitor device may open a second procedure session corresponding to the second device identifier information obtained from the first device identifier of the first visualisation device. Hence, a procedure session may be created for each individual visualisation device. A procedure session may be implemented by creating a folder in the file system of the monitor device, such that image files and video sequences obtained from a visualisation device can be stored in the folder corresponding to the visualisation device. Hence, association of an image file to a procedure session may be implemented by storing the image file in the folder of the procedure session. Opening a procedure session may further comprise creating a log, registering the time and date for initiating the procedure, registering information about the visualisation device, registering software version and/or other information.

In opening the procedure session, the monitor device may determine, based on the device identifier information, whether the visualisation device has been previously connected to the monitor device. For example, in accordance with determining that the visualisation device has previously been connected to the monitor device, the monitor device may reopen the procedure session corresponding to the device identifier information; and/or in accordance with determining that the visualisation device has not previously been connected to the monitor device, the monitor device may create the procedure session, e.g. a new procedure session, corresponding to the device identifier information.

A folder icon is displayed, e.g. by the monitor device and/or with the touch sensitive display of the monitor device. The folder icon may be displayed within a background portion of the graphical user interface. The background portion may be a portion other than the first portion of the graphical user interface. For example, the background portion may be the second portion or the fourth portion of the graphical user interface. The folder icon may comprise a visual representation of a count of stored files stored during the procedure session.

A first user input corresponding to selection of the image capture button of the one or more actionable items are received and/or detected on the touch sensitive display e.g. while displaying the live representation within the first portion and the one or more actionable items in the second portion. The monitor device, e.g. with the touch sensitive display, may detect the first user input. In response to detection of the first user input, the monitor device may store a first image file corresponding to the image data received when the first user input was detected. The monitor device may further, in response to the first user input, associate the first image file with the procedure session. For example, the monitor device may position the first image file in the folder of the procedure session, e.g. the procedure session corresponding to the connected visualisation device.

Also, in response to detection of the first user input a first representation of a still image corresponding to the stored first image file is displayed, e.g. within the background portion of the graphical user interface, e.g. by the monitor device and/or with the touch sensitive display of the monitor device. Providing a representation of the captured still image notifies the operator that an image is stored and provides an example of the stored image allowing the operator to quickly confirm that the image shows what he/she intended to capture.

After a predetermined delay after detection of the first user input an animation of transitioning the first representation to the folder icon is displayed, e.g. by the monitor device and/or with the touch sensitive display of the monitor device. Thereby, the operator may be visually notified that the captured image is stored and placed in the folder represented by the folder icon. Thus, the operator is made aware where he/she is able to retrieve the just captured image.

The present inventors have investigated the preferable time delay and found that optimally, the predetermined delay is between 1-8 seconds, such as between 3-7 seconds, such as between 4-6 seconds, such as 5 seconds or between 1.5-3 seconds, such as 1.5 seconds or such as 2 seconds. The present inventors have also investigated the preferable duration of the animation and found that optimally, the animation may have a duration between 100-1500 ms, such as between 300-1000 ms, such as between 300-800 ms, such as between 300-600 ms or between 500-700 ms. For example, the duration of the animation may be 400 ms, 500 ms or 600 ms. The duration of the animation may be a fraction of the predetermined delay, such as between 1/7 to 1/13 of the predetermined delay, such as between 1/8-1/12 of the predetermined delay, such as between 1/9-1/11 of the predetermined delay, such as 1/10 of the predetermined delay.

Also, in response to detection of the first user input, alternatively after display of the animation of transitioning the first representation to the folder icon, display of the visual representation of the count of stored files of the folder icon may be updated, e.g. including increasing the count of stored files.

After detection of the first user input, a second user input corresponding to selection of the image capture button may be received and/or detected. The monitor device may be adapted to detect the second user input. In response to detection of the second user input the monitor device may store a second image file corresponding to the image data received when the second user input was detected. The monitor device may further, in response to the second user input, associate the second image file with the procedure session. For example, the monitor device may position the second image file in the folder of the procedure session, e.g. the procedure session corresponding to the connected visualisation device.

Also, in response to detection of the second user input a second representation of a still image corresponding to the stored second image file may be displayed, e.g. within the background portion of the graphical user interface, e.g. by the monitor device and/or with the touch sensitive display of the monitor device. After the predetermined delay after detection of the second user input an animation of transitioning the second representation to the folder icon may be displayed, e.g. by the monitor device and/or with the touch sensitive display of the monitor device.

Also, in response to detection of the second user input, alternatively after display of the animation of transitioning the second representation to the folder icon, display of the visual representation of the count of stored files of the folder icon may be updated, e.g. including increasing the count of stored files.

By providing representations, animations and folder icons associated with the captured still image(s) within the background portion, e.g. within the second portion or the fourth portion of the graphical user interface, the indications related to the capturing of images may be provided simultaneously with showing the live representation of the image data, within the first portion of the graphical user interface, and without interfering with the live representation of the image data.

The monitor device may be adapted to establish connection to a second visualisation device, e.g. while a first visualisation device is still connected, or after connection with the first visualisation device has been disconnected. In establishing connection to the second visualisation device, the monitor device may obtain second device identifier information from the second device identifier of the second visualisation device. In response to establishing the connection to the second visualisation device, the monitor device may open a second procedure session, e.g. corresponding to the second device identifier information.

A live representation of the second image data generated by the second image sensor of the second visualisation device may be displayed. The live representation of the second image data may be displayed in the first portion of the graphical user interface.

A second folder icon may be displayed, e.g. by the monitor device, such as with the touch sensitive display of the monitor device. The second folder icon may be displayed within the background portion of the graphical user interface, e.g. within the second portion or the fourth portion of the graphical user interface. The second folder icon may comprise a visual representation of a count of stored files stored during the second procedure session. Display of the second folder icon may replace display of the folder icon associated with the previously described visualisation device, e.g. the first visualisation device, e.g. if the connection between the monitor device and previously described visualisation device has been disconnected.

While the second visualisation device is connected to the monitor device and/or while displaying the live representation of the second image data within the first portion and the one or more actionable items in the second portion, a third user input corresponding to selection of the image capture button may be received and/or detected. The monitor device may be adapted to detect the third user input, e.g. with the touch sensitive display. In response to detection of the third user input, the monitor device may store a third image file corresponding to the second image data received when the third user input was detected. The monitor device may further, in response to the third user input, associate the third image file with the second procedure session. For example, the monitor device may position the third image file in the second folder of the second procedure session, e.g. the procedure session corresponding to the connected second visualisation device.

Also, in response to detection of the third user input a third representation of a still image corresponding to the stored third image file may be displayed, e.g. within the background portion of the graphical user interface, e.g. by the monitor device and/or with the touch sensitive display of the monitor device. After the predetermined delay after detection of the third user input an animation of transitioning the third representation to the second folder icon may be displayed, e.g. by the monitor device and/or with the touch sensitive display of the monitor device.

Also, in response to detection of the third user input, alternatively after display of the animation of transitioning the third representation to the second folder icon, display of the visual representation of the count of stored files of the second folder icon may be updated, e.g. including increasing the count of stored files.

The video capture button may be displayed in a first appearance. A fourth user input corresponding to selection of the video capture button may be received and/or detected. The monitor device, e.g. with the touch sensitive display, may be adapted to detect the fourth user input. In response to detection of the fourth user input the monitor device may change the appearance of the video capture button to a second appearance. Furthermore, also in response to detection of the fourth user input, the monitor device may start collection of image data received from the image sensor (corresponding to the image sensor of the connected visualisation device, e.g. the first visualisation device and/or the second visualisation device) and temporarily stores the data in memory.

After detection of the fourth user input, a fifth user input corresponding to selection of the video capture button may be received and/or detected. The monitor device may be adapted to detect the fifth user input, e.g. with the touch sensitive display. In response to detection of the fifth user input, the monitor device changes the appearance of the video capture button to the first appearance. Also in response to detection of the fifth user input, the monitor device stores a first video data file corresponding to the image data received between detection of the fourth user input and the fifth user input. Also in response to detection of the fifth user input, the monitor device associates the first video data file with the procedure session (corresponding to the open procedure session according to the connected visualisation device, e.g. the procedure session may be the procedure session corresponding to the first visualisation device and/or the second procedure session corresponding to the second visualisation device).

Also, in response to detection of the fifth user input a fourth representation corresponding to a frame of the stored first video data file may be displayed, e.g. within the background portion of the graphical user interface, e.g. by the monitor device and/or with the touch sensitive display of the monitor device. After the predetermined delay after detection of the fifth user input an animation of transitioning the fourth representation to the folder icon (e.g. the folder icon associated with the first visualisation device or the second folder icon associated with the second first visualisation device) may be displayed, e.g. by the monitor device and/or with the touch sensitive display of the monitor device.

Also, in response to detection of the fifth user input, alternatively after display of the animation of transitioning the fourth representation to the folder icon, display of the visual representation of the count of stored files of the folder icon may be updated, e.g. including increasing the count of stored files.

A sixth user input corresponding to selection of the folder icon may be received and/or detected. The monitor device may be adapted to detect the sixth user input, e.g. with the touch sensitive display. In response to detection of the sixth user input, a first plurality of representations corresponding to a first plurality of stored image files stored during the procedure session may be displayed, e.g. by the monitor device and/or with the touch sensitive display of the monitor device. The first plurality of representations may be displayed within the background portion of the graphical user interface, e.g. within the fourth portion of the graphical user interface.

A seventh user input may be received and/or detected. The seventh user input may correspond to selection of a primary representation of the first plurality of representations displayed in response to detection of the sixth user input. The primary representation may correspond to a primary stored image file. The monitor device may be adapted to detect the seventh user input. In response to detection of the seventh user input an enlarged representation of the primary stored image file may be displayed, e.g. within the first portion of the graphical user interface, e.g. by the monitor device and/or with the touch sensitive display of the monitor device. Furthermore, in response to detection of the seventh user input thumbnail representations of a second plurality of the stored image files stored during the procedure session may be displayed, e.g. within the first portion of the graphical user interface, e.g. by the monitor device and/or with the touch sensitive display of the monitor device.

Further in response to detection of the sixth user input, a session overview icon may be displayed, e.g. within the background portion of the graphical user interface, e.g. by the monitor device and/or with the touch sensitive display of the monitor device. An eighth user input corresponding to selection of the session overview icon may be received and/or detected. The monitor device may be adapted to detect the eighth user input, e.g. with the touch sensitive display. In response to detection of the eighth user input a third plurality of representations corresponding to a third plurality of stored image files stored during the procedure session may be displayed, e.g. within the first portion of the graphical user interface, e.g. by the monitor device and/or with the touch sensitive display of the monitor device. The third plurality of representations may be displayed in a grid-like pattern.

Also, e.g. in response to detection of the eighth user input general information of the procedure session may be displayed, e.g. in the second portion of the graphical user interface, e.g. by the monitor device and/or with the touch sensitive display of the monitor device. Also, e.g. in response to detection of the eighth user input, a note field may be displayed, e.g. within the fourth portion of the graphical user interface, e.g. by the monitor device and/or with the touch sensitive display of the monitor device.

A ninth user input corresponding to selection of the note field may be received and/or detected. The monitor device may be adapted to detect the ninth user input. In response to detection of the ninth user input a virtual keyboard may be displayed, e.g. within the first portion of the graphical user interface and optionally extending into the second portion and/or fourth portion of the graphical user interface. The virtual keyboard may be displayed by the monitor device and/or with the touch sensitive display of the monitor device. The virtual keyboard is configured for entering text in the note field.

A sequence of keyboard user inputs corresponding to typing of a text using the displayed virtual keyboard may be displayed. The monitor device may be adapted to detect the sequence of keyboard user inputs, e.g. with the touch sensitive display. In response to detection of the sequence of keyboard user inputs, a corresponding text string may be displayed in the note field. A tenth user input indicative of accept of the text typed using the displayed virtual keyboard may be received and/or detected, e.g. the user may press an accept button. The monitor device may be adapted to detect the tenth user input, and in response to detection of the tenth user input, the monitor device may store the typed text and associate the typed text as a note for the procedure session.

The monitor device may be adapted to detect disconnection of a visualisation device. In response to disconnection of the visualisation device from the monitor device, e.g. in response to a detection of disconnection of the visualisation device the same as in response to detection of the sixth user input as described above may be displayed, e.g. by the monitor device and/or with the touch sensitive display of the monitor device, i.e. displaying a first plurality of representations corresponding to a first plurality of stored image files stored during the procedure session, e.g. wherein the procedure session corresponds to the just removed visualisation device. The first plurality of representations may be displayed within the background portion of the graphical user interface, e.g. within the fourth portion of the graphical user interface. Furthermore, also in response to disconnection of the visualisation device from the monitor device the session overview icon may be displayed within the background portion of the graphical user interface.

The one or more actionable menu items, e.g. displayed in the third portion of the graphical user interface, may comprise an archive menu item. An eleventh user input corresponding to selection of the archive menu item may be received and/or detected. The monitor device may be adapted to detect the eleventh user input, e.g. with the touch sensitive display. In response to detection of the eleventh user input, a primary archive menu associated with the archive menu item may be displayed, e.g. within the fourth portion of the graphical user interface. The primary archive menu may be displayed by the monitor device and/or with the touch sensitive display of the monitor device. The primary archive menu may comprise one or more primary actionable archive items, e.g. including a first primary actionable archive item and/or a second primary actionable archive item.

While displaying the primary archive menu, a twelfth user input corresponding to selection of the first primary actionable archive item may be received and/or detected. The monitor device may be adapted to detect the twelfth user input. In response to detection of the twelfth user input a secondary archive menu associated with the first primary actionable archive item may be displayed e.g. by the monitor device and/or with the touch sensitive display of the monitor device. The secondary archive menu may be displayed in the first portion of the graphical user interface, optionally extending into the second portion and/or the fourth portion of the graphical user interface.

The monitor device may comprise features, which should be restricted. Therefore, the monitor device may support authentication of a user allowing access to restricted features. Accordingly, the monitor device may operate an authorised state and/or a non-authorised state.

In accordance with the monitor device operating in an authorised state, the secondary archive menu may comprise a list of stored procedure sessions, e.g. a complete list of all procedures stored in the memory of the monitor device. In accordance with the monitor device operating in a non-authorised state and a setting to require authorisation is activated, the secondary archive menu may comprise an empty list or a list of a subset of the stored procedure sessions. The subset may, e.g., be procedure sessions recorded on the current day or the last recorded session. In accordance with the monitor device operating in a non-authorised state and a setting to require authorisation is deactivated, the secondary archive menu may comprise the list of stored procedure sessions, e.g. the complete list of all procedures stored in the memory of the monitor device.

While displaying the secondary archive menu comprising the list of stored procedure sessions or the list of a subset of the stored procedure sessions, a thirteenth user input, corresponding to selection of a first stored procedure session of the list of stored procedure sessions or the list of a subset of the stored procedure sessions, may be received and/or detected. The monitor device may be adapted to detect the thirteenth user input, e.g. with the touch sensitive display. In response to detection of the thirteenth user input, a fourth plurality of representations corresponding to a fourth plurality of stored image files stored during the first stored procedure session may be displayed, e.g. in the first portion of the graphical user interface. The monitor device and/or the touch sensitive display of the monitor device may display the fourth plurality of representations.

Also in response to detection of the thirteenth user input, general information of the first stored procedure session may be displayed, e.g. in the fourth portion of the graphical user interface. Also in response to detection of the thirteenth user input a note field may be displayed, e.g. in the fourth portion of the graphical user interface.

The graphical user interface and/or the graphical user interface contents displayed in response to the thirteenth user input may correspond to the graphical user interface and/or the graphical user interface contents displayed in response to the eighth user input, if the procedure session is the same.

A fourteenth user input, corresponding to selection of a select representation of the fourth plurality of representations may be received and/or detected. The select representation may correspond to a select stored image file. The monitor device may be adapted to detect the fourteenth user input. In response to detection of the fourteenth user input an enlarged representation of the select stored image file may be displayed, e.g. within the first portion of the graphical user interface, e.g. by the monitor device and/or with the touch sensitive display of the monitor device. Further in response to detection of the fourteenth user input, thumbnail representations of a fifth plurality of the stored image files stored during the first stored procedure session may be displayed, e.g. within the first portion of the graphical user interface, e.g. by the monitor device and/or with the touch sensitive display of the monitor device. The thumbnail representations may comprise a thumbnail representation of the select stored image file.

Also, in response to detection of the fourteenth user input image information associated with the select stored image file may be displayed, e.g. within the second portion of the graphical user interface, e.g. by the monitor device and/or with the touch sensitive display of the monitor device. The image information associated with the select stored image file may comprise information indicative of the visualisation device. The information indicative of the visualisation device may have been obtained from the device identifier information.

A fifteenth user input, corresponding to selection of a select thumbnail of the displayed thumbnail representations may be received and/or detected. The select thumbnail may correspond to a second select stored image file. The monitor device is further adapted to detect the fifteenth user input. In response to detection of the fifteenth user input an enlarged representation of the second select stored image file may be displayed, e.g. within the first portion of the graphical user interface, e.g. by the monitor device and/or with the touch sensitive display of the monitor device.

Also, in response to detection of the fifteenth user input, thumbnail representations of a sixth plurality of the stored image files stored during the first stored procedure session may be displayed, e.g. within the first portion of the graphical user interface, e.g. by the monitor device and/or with the touch sensitive display of the monitor device. The sixth plurality of the stored image files may be the same as the fifth plurality of the stored image files.

An export icon may be displayed, e.g. in response to detection of the fourteenth user input. A sixteenth user input corresponding to selection of the export icon may be received. The monitor device may be adapted to detect the sixteenth user input, e.g. with the touch sensitive display. In response to detection of the sixteenth user input the fourth plurality of representations corresponding to the fourth plurality of stored image files, may be displayed, e.g. in the first portion of the graphical user interface, e.g. by the monitor device and/or with the touch sensitive display of the monitor device. Each of the fourth plurality of representations may comprise a selection indicator, wherein the selection indicator of the select representation may be activated. Furthermore, in response to detection of the sixteenth user input, an export menu comprising an export confirm icon may be displayed, e.g. within the second portion and/or the fourth portion of the graphical user interface, e.g. by the monitor device and/or with the touch sensitive display of the monitor device.

Alternatively and/or additionally, the export icon may be displayed in response to detection of the seventh user input. In such case in response to detection of the user input corresponding to the selection of the export icon, the second plurality of representations corresponding to the second plurality of stored image files, may be displayed, e.g. in the first portion of the graphical user interface, e.g. by the monitor device and/or with the touch sensitive display of the monitor device. Each of the second plurality of representations may comprise a selection indicator, wherein the selection indicator of the primary stored image file may be activated. Similar to described above an export menu comprising an export confirm icon may be displayed.

Alternatively and/or additionally, the export icon may be displayed in response to detection of the eighth user input. In such case in response to detection of the user input corresponding to the selection of the export icon, the third plurality of representations corresponding to the third plurality of stored image files, may be displayed, e.g. in the first portion of the graphical user interface, e.g. by the monitor device and/or with the touch sensitive display of the monitor device. Each of the third plurality of representations may comprise a selection indicator, wherein none of the selection indicators are initially activated. Similar to described above an export menu comprising an export confirm icon may be displayed.

Alternatively and/or additionally, the export icon may be displayed in response to detection of the thirteenth user input. In such case in response to detection of the user input corresponding to the selection of the export icon, the fourth plurality of representations corresponding to the fourth plurality of stored image files, may be displayed, e.g. in the first portion of the graphical user interface, e.g. by the monitor device and/or with the touch sensitive display of the monitor device. Each of the fourth plurality of representations may comprise a selection indicator, wherein none of the selection indicators are initially activated. Similar to described above an export menu comprising an export confirm icon may be displayed.

After detection of the sixteenth user input, a seventeenth user input, corresponding to selection of one or more of the selection indicators, e.g. of the fourth plurality of representations, may be received and/or detected. The monitor device may be adapted to detect the seventeenth user input, e.g. with the touch sensitive display. In response to detection of the seventeenth user input, the selection indicators of the plurality of representations corresponding to the selected one or more selection indicators may be activated.

After detection of the sixteenth user input and/or after detection of the seventeenth user input, an eighteenth user input, corresponding to selection of the export confirm icon may be received and/or detected. The monitor device may be adapted to detect the eighteenth user input, e.g. with the touch sensitive display. In response to detection of the eighteenth user input stored image files corresponding to the selected one or more selection indicators may be transmitted to an auxiliary device, such as a USB-drive, or a remote server. For example, the monitor device may transmit the stored image files corresponding to the selected one or more selection indicators to the auxiliary device, e.g. via Bluetooth, USB, LAN, WiFi or any other suitable connection.

A deletion icon may be displayed, e.g. in response to detection of the fourteenth user input, the seventh user input, the eight user input, the thirteenth user input, the sixteenth user input, and/or the seventeenth user input. A nineteenth user input corresponding to selection of the deletion icon may be received and/or detected. The monitor device may be adapted to detect the nineteenth user input, e.g. with the touch sensitive display. In response to detection of the nineteenth user input, a confirmation dialogue indicative of potential deletion of one or more image files (e.g. corresponding to a displayed enlarged representation or stored image files having their selection indicators selected) may be displayed, e.g. by the monitor device and/or with the touch sensitive display of the monitor device.

A twentieth user input to the confirmation dialogue may be received. The monitor device may be adapted to detect the twentieth user input, e.g. with the touch sensitive display. In accordance with the twentieth user input being indicative of the user confirming deletion of the one or more image files, the one or more image files may be deleted, e.g. removed from memory. Furthermore, display of the enlarged representation may be replaced with an enlarged representation of a second image file. In accordance with the twentieth user input being indicative of the user cancelling deletion of the select stored image file, deletion of the one or more image file may be forgone. Furthermore, display of the enlarged representation may be maintained, e.g. within the first portion of the graphical user interface.

The deletion icon may be displayed in response to detection of the fourteenth user input. In this case, in response to detection of the nineteenth user input, a confirmation dialogue indicative of potential deletion of the select stored image file is displayed. In accordance with the twentieth user input being indicative of the user confirming deletion of the select stored image file the select stored image file is deleted. Furthermore, display of the enlarged representation of the select stored image file is replaced with an enlarged representation of a second select stored image file. In accordance with the twentieth user input being indicative of the user cancelling deletion of the select stored image file, deletion of the select stored image file is forgone. Furthermore, display of the enlarged representation of the select stored image file within the first portion of the graphical user interface is maintained.

### BRIEF DESCRIPTION OF THE FIGURES

Embodiments of the disclosure will be described in more detail in the following with regard to the accompanying figures. The figures show one way of implementing the present invention and are not to be construed as being limiting to other possible embodiments falling within the scope of the attached claim set.
Fig. 1 schematically illustrates an exemplary medical visualisation system,
Fig. 2 schematically illustrates an exemplary monitor device,
Fig. 3 schematically illustrates an exemplary monitor device,
Fig. 4 is a block diagram of an exemplary monitor device,
Figs. 5A-5H schematically illustrates exemplary user interactions with an exemplary graphical user interface,
Figs. 6A-6D schematically illustrates exemplary user interactions with an exemplary graphical user interface,
Figs. 7A-7F schematically illustrates exemplary user interactions with an exemplary graphical user interface,
Figs. 8A-8H schematically illustrates exemplary user interactions with an exemplary graphical user interface,
Figs. 9A-9K schematically illustrates exemplary user interactions with an exemplary graphical user interface,
Figs. 10A-10D schematically illustrates exemplary user interactions with an exemplary graphical user interface, and
Figs. 11A-11E schematically illustrates exemplary user interactions with an exemplary graphical user interface.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

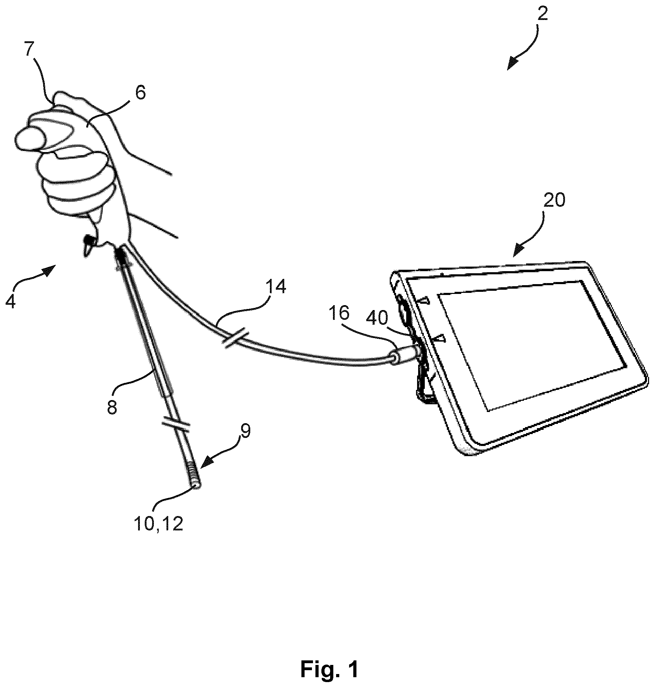

Fig. 1 schematically illustrates an exemplary medical visualisation system 2 comprising a visualisation device 4 and a monitor device 20. The visualisation device 4 has an image sensor 12, e.g. a CCD or a CMOS, configured to generate image data indicative of a view from the visualisation device 4. In the illustrated example, the visualisation device 4 is an endoscope comprising a handle 6 and an elongated flexible member 8, e.g. an insertion tube, extending from the handle 6 to a distal end 10. The image sensor 12 may be configured to generate image data indicative of a view from the distal end 10 of the elongated flexible member 8.

The visualisation device 4 may be connected to the monitor device 20. In the illustrated example, a device cable 14 extending from the handle 6 terminates in a device connector 16 connected to a connection port 40 of the monitor device 20. The monitor device 20 is operable to receive image data generated by the image sensor 12 of the visualisation device 4. For example, the monitor device 20 may receive image data generated by the image sensor 12 via the device cable 14, the connector 16 and connection port 40.

The handle 6 comprises a control button 7 adapted to receive an input in a first input direction and/or in a second input direction. The touch input in the first input direction on the control button 7 causes a distal portion 9 of the elongated flexible member 8 to bend in a first bending direction, e.g. via wires extending from the handle, through the elongated flexible member 8 to the distal portion 9. The touch input in the second input direction on the control button 7 causes the distal portion 9 of the elongated flexible member 8 to bend in a second bending direction. The first input direction and the second input direction may be opposite. The first bending direction and the second bending direction may be opposite. Bending the distal portion 9 of the elongated flexible member 8 may cause a movement of the distal end 10 and the image sensor 12 in a direction relative to the image sensor 12. Thereby, seeing an image generated by the image sensor 12, a direction, e.g. up or down, in the image may correspond to a respective input on the control button 7.

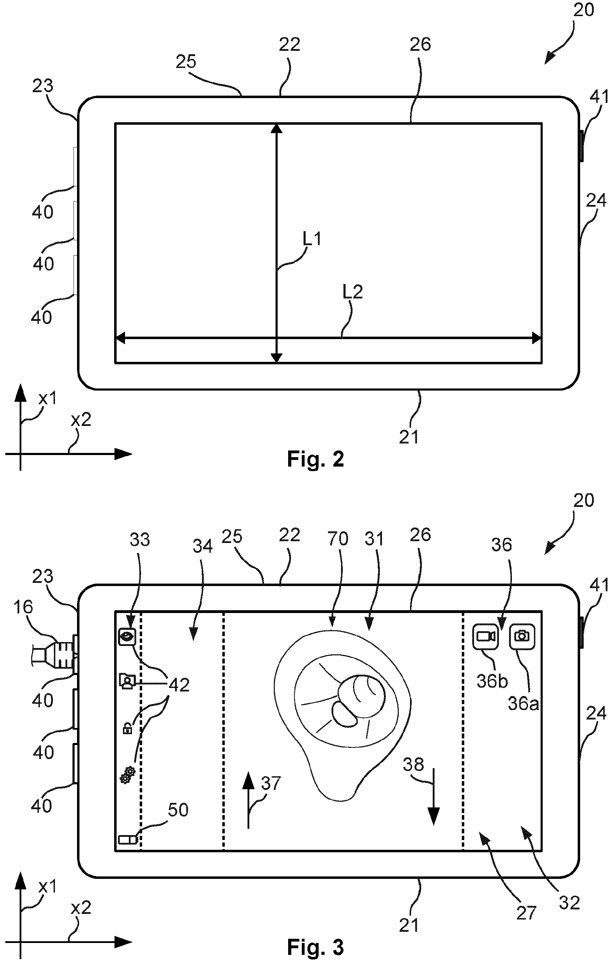

Fig. 2 schematically illustrates an exemplary monitor device 20, such as the monitor device 20 as illustrated in Fig. 1. The monitor device 20 comprises a first housing 25. The first housing 25 extends in a first direction x1 from a first housing side 21 to a second housing side 22 and in a second direction x2 perpendicular to the first direction x1 from a third housing side 23 to a fourth housing side 24. The monitor device comprises a touch sensitive display 26 accommodated in the first housing 25. The touch sensitive display 26 has a first length L1 in the first direction x1 and a second length L2 in the second direction x2. The second length L2 may be longer than the first length L1 as illustrated.

The monitor device may comprise one or more connection port(s) 40, such as three connection ports 40, as illustrated. The connection ports 40 may allow visualisation devices to be connected. The connection port(s) 40 may be arranged at the third housing side 23, as illustrated. Alternatively or additionally, connection port(s) 40 may be arranged at the fourth housing side 24.

The monitor device may comprise an on/off button 41, which may be provided on the fourth housing side 24, as illustrated.

Fig. 3 schematically illustrates an exemplary monitor device 20, such as the monitor device 20 as illustrated in Figs. 1-2. As illustrated a device connector 16 may be connected to a connection port 40.

The monitor device 20 may be provided with a graphical user interface 27. The graphical user interface 27 may be displayed with the touch sensitive display 26, and the user may interact with the graphical user interface 27, e.g. by means of providing touch inputs on the touch sensitive display 26.

The graphical user interface 27 is displayed with the touch sensitive display 26. The graphical user interface 27 comprises a plurality of non-overlapping portions 31, 32, 33, 34. Each of the portions 31, 32, 33, 34 extends substantially throughout the first length L1 in the first direction x1. The non-overlapping portions includes a first portion 31, a second portion 32, a third portion 33 and a fourth portion 34. The first portion 31 is arranged between the fourth portion 34 and the second portion 32 along the second direction x2. The fourth portion 34 is arranged between the third portion 33 and the first portion 31 along the second direction x2. The third portion 33 is arranged between a side of the first housing, e.g. the third housing side 23, and the fourth portion 34 along the second direction x2. The second portion 32 is arranged between another side of the first housing 25, e.g. the fourth housing side 24, and the first portion 31 along the second direction x2. The first portion 31 and the fourth portion 34 are arranged between the second portion 32 and the third portion 33 along the second direction.

The monitor device 20 displays a live representation 70 of the image data within the first portion 31 of the touch sensitive display 26. The first bending direction and the second bending direction of the distal portion 9 of the elongated flexible member 8, as described with respect to Fig. 1, may corresponds to a first image direction 37 and a second image direction 38 of the live representation 70, respectively. The first image direction 37 and the second image direction 38 may be parallel to the first direction x1, as illustrated. The first image direction 37 and the second image direction 38 may be opposite, as illustrated. Thereby, a user operating the control button 7 of visualisation device 2 may cause movement of the distal portion 9 of the elongated flexible member 8 to bend in a direction corresponding to the first image direction 37 or the second image direction 38 of the live representation 70.

The monitor device 20 displays with the touch sensitive display 26 one or more actionable items 36 within the second portion 32 of the graphical user interface 27. The actionable items 36 may comprise an image capture button 36a, e.g. for storing an image data file corresponding to the image data received when the image capture button 36a was activated. Alternatively or additionally, the actionable items 36 may comprise a video capture button 36b, e.g. for storing a video sequence of image data corresponding to the image data received when the video capture button 36b was activated.

The monitor device 20 displays with the touch sensitive display 26 one or more actionable menu items 42 within the third portion 33 of the graphical user interface 27. The actionable menu items 42 may, for example, comprise a login menu item for initiating a login procedure, a settings menu item for accessing a settings menu, an archive menu item for browsing an archive, and a default menu item for returning to a default view. Also a battery indicator 50 is displayed in the third portion 33.

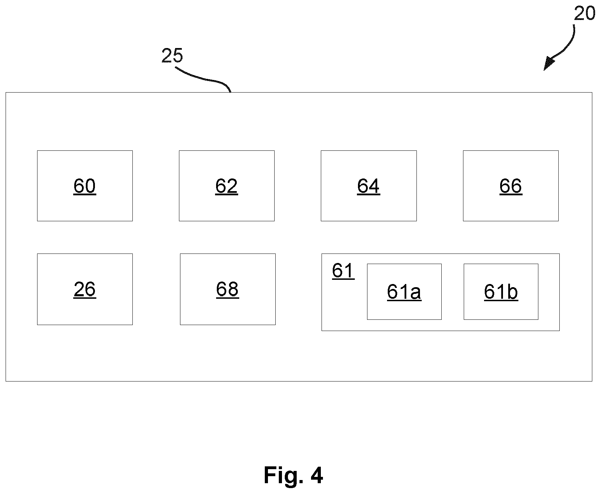

Fig. 4 is a block diagram of an exemplary monitor device 20, such as the monitor device 20 of the previous figures. The monitor device 20 comprises a processing unit 60 and memory 62. The memory 62 may comprise both volatile and non-volatile memory. The monitor device 20 also comprise an orientation sensor 64 for determining the orientation of the first housing 25 relative to gravity. The orientation sensor 64 may comprise one or more accelerometers and/or a gyroscope. The monitor device 20 comprises input/output module 66, such as for receiving image data from the image sensor 12 via connectors of visualisation device 4. The input/output module 66 may also comprise ethernet connector, WiFi transceiver, Bluetooth transceiver, video connectors, USB ports etc., and respective controllers. The monitor device 20 also comprises the touch sensitive display 26 as described earlier. The monitor device 20 may display information, graphical user interface objects, images, buttons etc, with the touch sensitive display 27. The monitor device 20 also comprises a microphone 68. The monitor device 20 comprises a power unit 61 for powering the monitor device 20. The power unit 61 may comprise a rechargeable battery 61a. The power unit 61 may comprise a power connection 61b for connecting the power unit 61 to an external power supply, such as a conventional AC power socket. The components of the monitor device 20 may be interconnected by buses or signal lines. Some or all of the components of the monitor device may be accommodated in the first housing 25 as illustrated. However, alternatively some of the components, e.g. the processing unit 60, the memory 62, input/output module 66 and/or the power unit 61 may be accommodated in a second housing of the monitor device 20.

The power unit 61 may comprise components for, e.g. indirectly measuring, capacity of the rechargeable battery 61a. For example, the power unit 61 may comprise a voltage gauge to measure the voltage of the rechargeable battery 61a. Based on the measured voltage, the remaining capacity of the rechargeable battery 61a may be estimated, e.g. by the processing unit 60. The power unit 61 may also comprise components for measuring power consumption of the monitor device 20. For example, the power unit 61 may comprise a power meter to measure the rate at which the monitor device 20 consumes power from the rechargeable battery 61a. The voltage gauge may be a low current consumption integrated circuit or a resistor coupled in parallel with the battery. A current sensor may be provided and the power may be computed as the product of the voltage and current. Additionally, an integrated circuit may be provided that includes a voltage gauge and a current sensor, and which outputs a power value in digital form.

The monitor device 20 may display content with the touch sensitive display 26. For example, the monitor device 20 may display content by the processing device 60 transmitting instructions to the touch sensitive display 26 indicative of the content to be displayed. The monitor device 20 may receive user input with the touch sensitive display 26. Particularly, the monitor device 20 may detect user inputs with the touch sensitive display 26. For example, a user providing a touch input on the touch sensitive display 26 causes a change in one or more electrical parameters of the touch sensitive display 26 indicative of at least the location of the touch input. Information of the touch input is transmitted from the touch sensitive display 26 to the processing unit 60, and the processing unit 60 may determine whether the touch input corresponds to an action to perform, e.g. whether the location of the touch input corresponds to the location of a soft-button displayed at the touch sensitive display.

The user may interact with the monitor device 20 via the graphical user interface 27 by providing user inputs, e.g. by means of providing touch inputs on the touch sensitive display 26, and the monitor device 20 may detect such user inputs with the touch sensitive display 26. A touch input, e.g. a single tap, double tap, swipe or similar, and the location of the touch input on the touch sensitive display 26 is registered by the touch sensitive display 26, which transmits information of the touch input (e.g. including type of touch (double tap, single tap, swipe, etc.) and/or location of the touch) to the processing unit 60 of the monitor device 20. The processing unit 60 interprets the information received and determines whether the touch input corresponds to activation of an action, e.g. whether the touch input correspond to activation of a button displayed with the touch sensitive display 27 at the location of the touch input. In response to a determination that the touch input corresponds to activation of an action, the processing unit 60 performs the respective action.

For example, with reference to Figs. 3 and 4, to capture an image corresponding to the presently shown live representation 70, e.g. corresponding to the image data received from the image sensor, the user may tap the image capture button 36a. The tap and the location of the tap is registered by the touch sensitive display 26, which transmits the information of the tap to the processing unit 60 of the monitor device 20. The processing unit 60 interprets the information received and determines that the user tapped the location corresponding to the image capture button 36a. In response thereto, the processing unit 60 stores, in memory 62 an image data file corresponding to the image data received.

In further reference to Figs. 3 and 4, to capture a video sequence corresponding to the shown live representation 70 over a period of time, e.g. corresponding to the image data received from the image sensor over a period of time, the user may tap the video capture button 36b. The tap and the location of the tap is registered by the touch sensitive display 26, which transmits the information of the tap to a processing unit 60 (see Fig. 4) of the monitor device 20. The processing unit 60 interprets the information received and determines that the user tapped the location corresponding to the video capture button 36b. In response thereto, the processing unit 60 starts collection of image data received from the image sensor 12 and temporarily stores the data in memory 62. To stop the recording, the user may tap the video capture button 36b again. The processing unit 60 determines, based on the signal received from the touch sensitive display 26, that that the user tapped the video capture button 36b and stops collecting image data received from the image sensor 12. The processing unit 60 read the temporarily stored data from the memory 62 and create a complete video sequence based thereon, which the processing unit 60 stores in the memory 62.

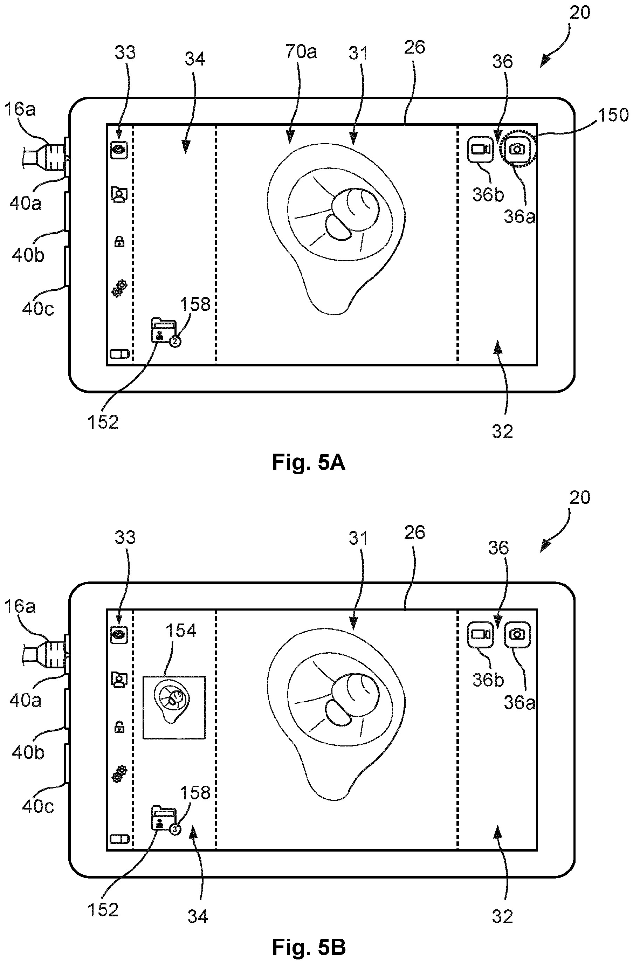

Figs. 5A-5H schematically illustrates exemplary user interactions with a graphical user interface of a monitor device 20, such as the monitor device 20 of any of the previous figures.

In Fig. 5A, as illustrated that a first visualisation device (not shown) has been connected to the monitor device 20, by a first device connector 16a of the first visualisation device being received at the first connection port 40a. A first live representation 70a of first image data generated by a first image sensor of the first visualisation device is displayed within the first portion 31 of the graphical user interface.

When a visualisation device is connected, e.g. when the monitor device 20 and/or the processing unit of the monitor device detects connection of a visualisation device, device identifier information from a device identifier of the respective visualisation device may be obtained. For example, the visualisation device may be fitted with an EPROM (alternatively a QR code, RFID tag, NFC etc may be used), which the monitor device 20 is able to read. For example, the processing unit of the monitor device may execute a process for interrogating the device identifier, via the device connector and connection port. The EPROM may store information of the visualisation device, e.g. a serial number of the visualisation device, which may uniquely identify the visualisation device. Also the device identifier information may be indicative of the type of visualisation device, e.g. whether it is an endoscope or a laryngoscope, brand of the visualisation device, production version, batch number etc.

In response to detecting connection of the visualisation device, and after obtaining the device identifier information, the monitor device may open (create or reopen, depending on whether the visualisation device has previously been connected) a procedure session corresponding to the device identifier information. For example, a first procedure session may be opened corresponding to the first device identifier information obtained from the first visualisation device, and a second procedure session may be opened corresponding to a second device identifier information obtained from a second visualisation device, if connected. The procedure sessions may be unique, and therefore reconnecting a previously connected visualisation device may cause the monitor device 20 to reopen a previously created session. A procedure session may be implemented by creating a folder in the file system of the monitor device 20, e.g. within the memory of the monitor device 20. Image files and video sequences obtained with a particular visualisation device may be stored in the folder corresponding to the visualisation device.

Thus, the monitor device 20 in opening the procedure session may determine, e.g. based on the device identifier information, whether the visualisation device has been previously connected to the monitor device. Accordingly, if determined that the visualisation device has previously been connected to the monitor device 20, the monitor device 20 reopens the procedure session corresponding to the device identifier information, and if determined that the visualisation device has not previously been connected to the monitor device 20, the monitor device 20 creates a new procedure session corresponding to the device identifier information.

The session, and the folder of images/videos, may be based on the device identifier information of the attached visualisation device. Hence, when attaching a new endoscope a new folder is created, and if reattaching a scope that was previously attached the previously created folder is reopened, and additionally captured still images or videos are saved to this existing folder. Hence, if an endoscope is pulled out by accident and inserted again, captured images/videos are not saved into a new folder, but organised in the same folder.

A folder icon 152 is displayed within a background portion (e.g. second portion 32 and/or fourth portion 34) of the graphical user interface. In the present example, the folder icon 152 is displayed within the fourth portion. Alternatively, the folder icon 152 may be shown in the second portion 32. The folder icon 152 comprises a visual representation 158 of a count of stored files, e.g. stored during the procedure session.

As illustrated in Fig. 5A, a user may provide a first user input 150 corresponding to selection of the image capture button 36a. The monitor device 20, e.g. with the touch sensitive display 26, is adapted to detect the first user input 150. In response to detection of the first user input 150, the monitor device stores a first image file corresponding to the image data received when the first user input 150 was detected, e.g. corresponding to the live representation 70a being displayed in the first portion 31. The monitor device 20 associates the first image file with the procedure session. For example, the monitor device 20 may store the first image file in the folder corresponding to the procedure session.

Furthermore, as illustrated in Fig. 5B, the monitor device, in response to detection of the first user input 150 and storing of the first image file displays within the background portion of the graphical user interface, e.g. the fourth portion 34 as illustrated, a first representation 154 of a still image corresponding to the stored first image file. Thereby, the operator is notified that the monitor device 20 has stored an image, and the operator is further provided with an example of the stored image, e.g. to allow the operator to quickly confirm that the image does show what he/she intended it to show.

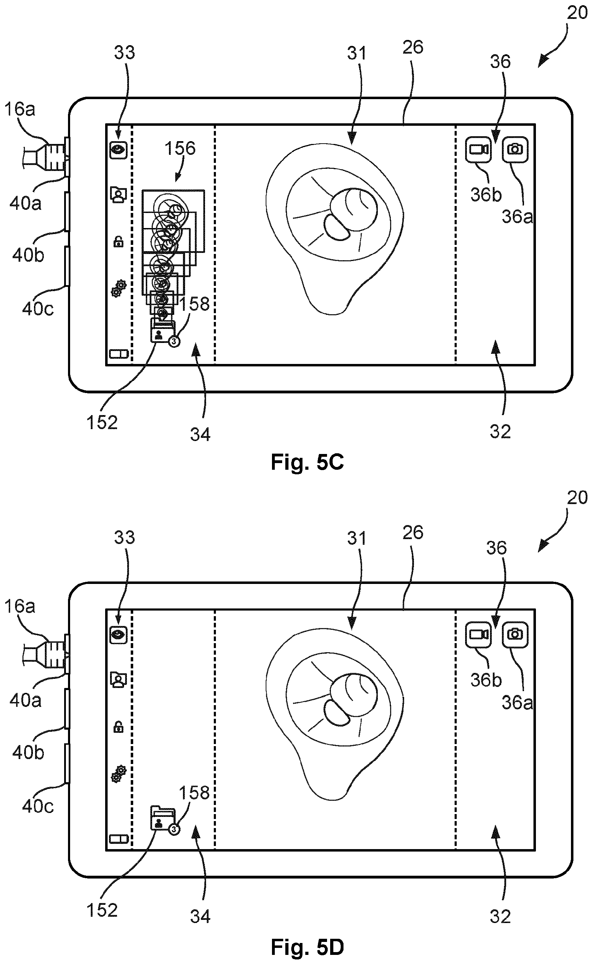

As shown in Fig. 5C, after a predetermined delay after detection of the first user input, the monitor device displays an animation 156 of transitioning the first representation 154 to the folder icon 152. The predetermined delay may be between 1-5 seconds, such as between 1.5-3 seconds, such as 1.5 seconds or such as 2 seconds. Thus, the operator is visually notified that the captured image is stored and is placed in the folder represented by the folder icon 152. The animation 156 may have a duration between 100-1500 ms, such as between 300-1000 ms, such as between 300-600 ms, such as 400 ms or 500 ms.

Furthermore, also in response to detection of the first user input 150 and storing of the first image file (cf. Fig. 5B), the display of the visual representation 158 of the count of stored files stored during the procedure session is updated by increasing the count of stored files. Alternatively, the display of the visual representation 158 of the count of stored files stored during the procedure session may be updated by increasing the count of stored files after display of the animation 156, e.g. in the transition between Fig 5C and Fig. 5D.

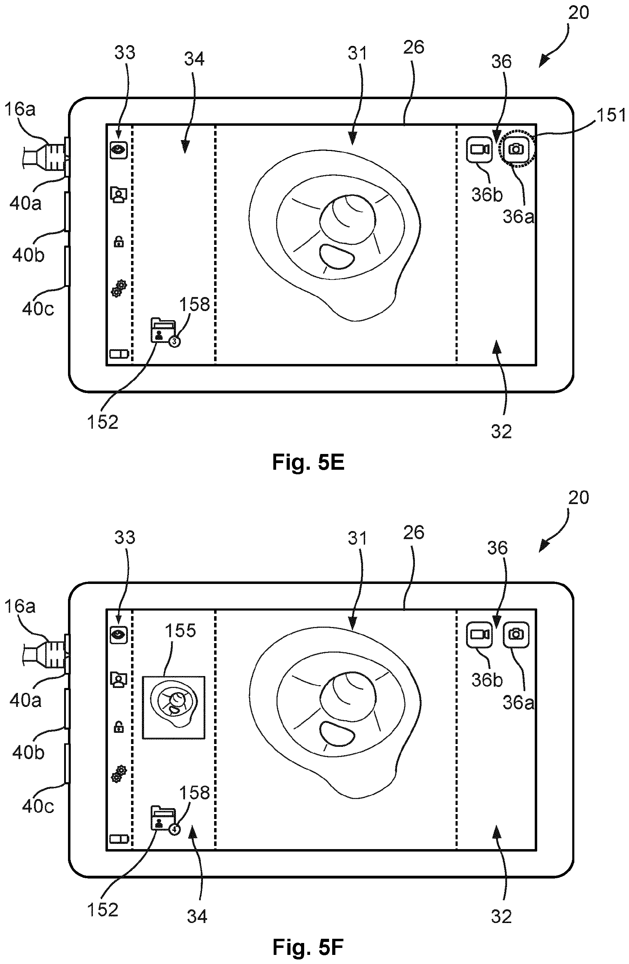

Figs. 5E-5H, shows capturing of a second image file after having stored the first image file as explained with reference to Figs. 5A-5D.

As illustrated in Fig. 5E, a user may provide a second user input 151 corresponding to selection of the image capture button 36a. In response to detection of the second user input 151, the monitor device stores a second image file corresponding to the image data received when the second user input 151 was detected, e.g. corresponding to the live representation 70a being displayed in the first portion 31. The monitor device 20 associates the second image file with the procedure session, e.g., the monitor device 20 may store the second image file in the folder corresponding to the procedure session, i.e. in the same folder as the first image file.

As illustrated in Fig. 5F, the monitor device, in response to detection of the second user input 151 and storing of the second image file displays within the background portion of the graphical user interface, e.g. the fourth portion 34 as illustrated, a second representation 155 of a still image corresponding to the stored second image file.

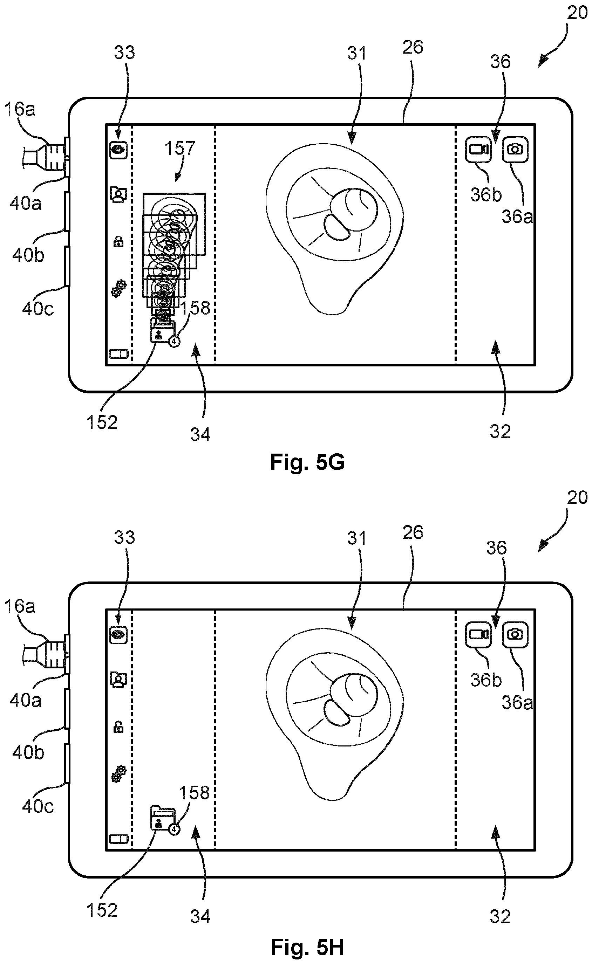

As shown in Fig. 5G after the predetermined delay after detection of the second user input 151, the monitor device displays an animation 157 of transitioning the second representation 155 to the folder icon 152. The animation 157 may have a duration between 100-1500 ms, such as between 300-1000 ms, such as between 300-600 ms, such as 400 ms or 500 ms.

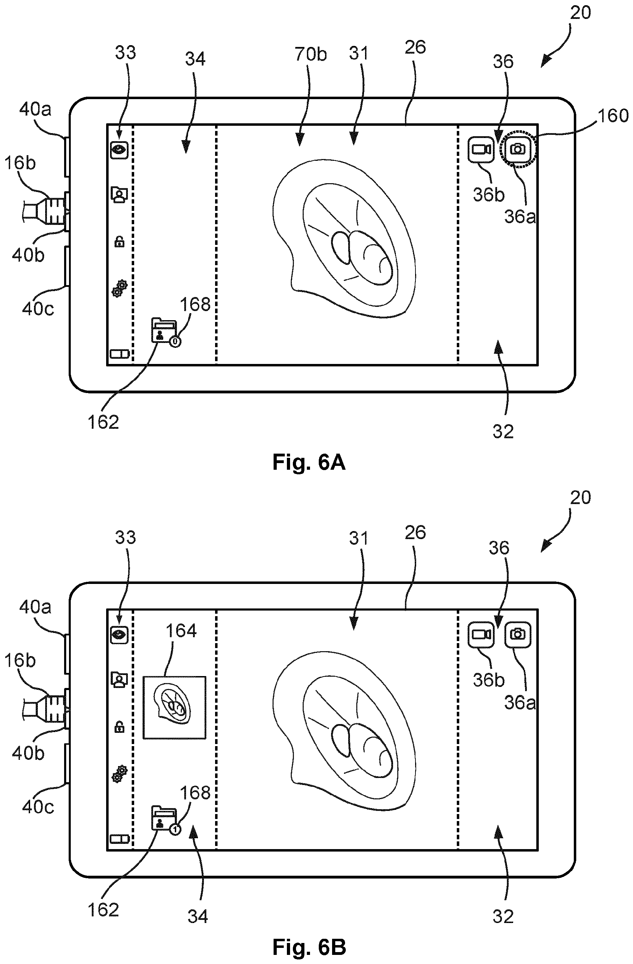

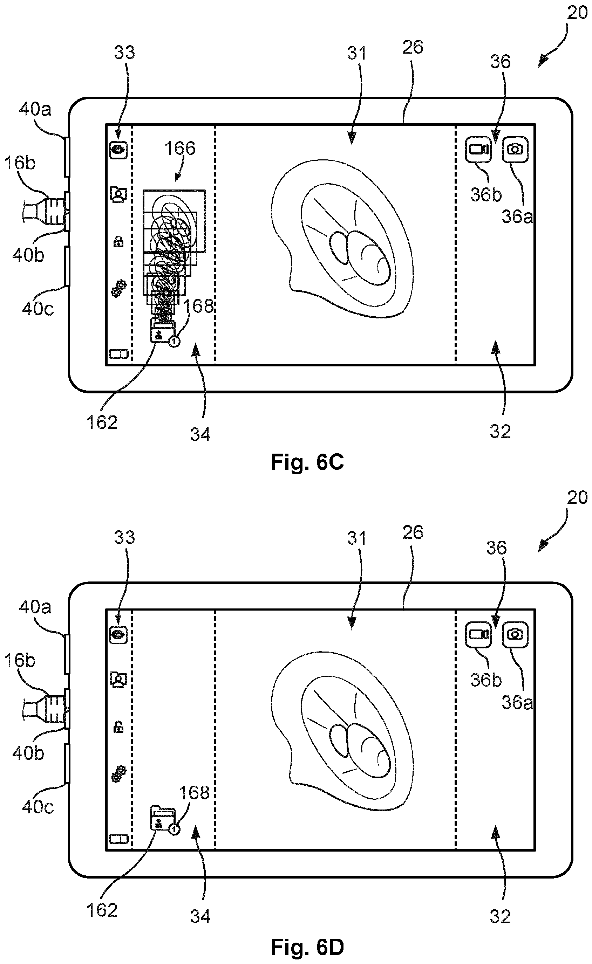

Figs. 6A-6D schematically illustrates exemplary user interactions with a graphical user interface of a monitor device 20, such as the monitor device 20 of any of the previous figures.

As seen in Fig. 6A, the first visualisation device, which was connected to the monitor device in Figs. 5A-5H, as illustrated by device connector 16a has been disconnected. Instead a second visualisation device having device connector 16b has been connected to the monitor device 20 via the second connection port 40b. The second visualisation device could have been equally connected to the first connection port 40a.

As explained above, when a visualisation device is connected, device identifier information from a device identifier of the respective visualisation device may be obtained, and the monitor device 20 may open a procedure session based on the device identifier information. Thus, in establishing connection to the second visualisation device, including obtaining second device identifier information from a second device identifier of the second visualisation device, the monitor device 20 may open a second procedure session corresponding to the second device identifier information, e.g. different from the procedure session as described with respect to Figs. 5A-5H.

Accordingly, the monitor device 20, as illustrated in Fig. 6A, displays a live representation 70b of second image data generated by a second image sensor of the second visualisation device, and a folder icon 162 is displayed within a background portion (e.g. second portion 32 and/or fourth portion 34) of the graphical user interface. In the present example, the folder icon 162 is displayed within the fourth portion. Alternatively, the folder icon 162 may be shown in the second portion 32. The folder icon 162 comprises a visual representation 168 of a count of stored files, e.g. stored during the second procedure session. As the second procedure session is different from the procedure session of Figs. 5A-5H, because the visualisation device is different, the visual representation 168 may have a different count than the visual representation 158 of Figs. 5A-5H.

As illustrated in Fig. 6A, a user may provide a third user input 160 corresponding to selection of the image capture button 36a. The monitor device 20, e.g. with the touch sensitive display 26, is adapted to detect the third user input 160. In response to detection of the third user input 160, the monitor device stores a third image file corresponding to the second image data received when the third user input 160 was detected, e.g. corresponding to the live representation 70b being displayed in the first portion 31. The monitor device 20 associates the third image file with the second procedure session. For example, the monitor device 20 stores the third image file in the folder corresponding to the second procedure session.

As illustrated in Fig. 6B, the monitor device 20, in response to detection of the third user input 160 and storing of the third image file displays within the background portion of the graphical user interface, e.g. the fourth portion 34 as illustrated, a third representation 164 of a still image corresponding to the stored third image file.

As shown in Fig. 6C, after a predetermined delay after detection of the third user input, the monitor device displays an animation 166 of transitioning the third representation 164 to the folder icon 162. The predetermined delay may be the same delay as explained with respect to Fig. 5C. The animation 166 may have a duration like the duration of the animation 156 as explained with respect to Fig. 5C.

Furthermore, also in response to detection of the third user input 160 and storing of the third image file (cf. Fig. 6B), the display of the visual representation 168 of the count of stored files stored during the second procedure session is updated by increasing the count of stored files.

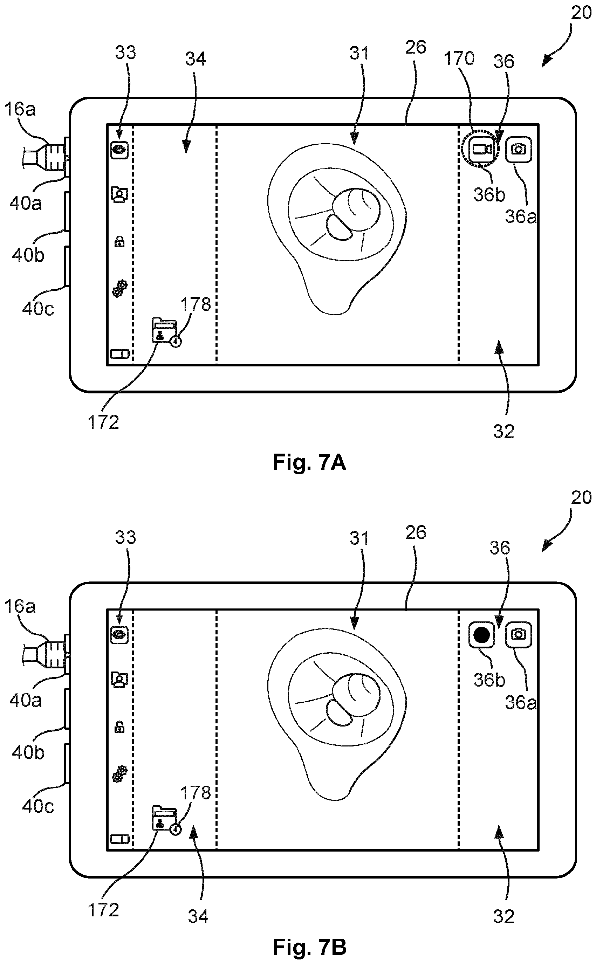

Figs. 7A-7F schematically illustrates exemplary user interactions with a graphical user interface of a monitor device 20, such as the monitor device 20 of any of the previous figures. The exemplary graphical user interface of Fig 7A may follow, e.g., after the exemplary graphical user interface of Fig. 5H. Thus, the folder icon 172 illustrated in Figs. 7A-7F may correspond to the folder icon 152 of Figs. 5A-5H, and the procedure session as referred to with respect to Figs. 7A-7F may be the same procedure session as explained in relation to Figs. 5A-5H.

As illustrated in Fig. 7A, a user may provide a fourth user input 170 corresponding to selection of the video capture button 36b. The video capture button 36b may initially be displayed in a first appearance, as illustrated in Fig. 7A. The monitor device 20, e.g. with the touch sensitive display 26, is adapted to detect the fourth user input 170. In response to detection of the fourth user input 170, the monitor device changes the appearance of the video capture button 36b to a second appearance, as illustrated in fig. 7B. Furthermore, also in response to detection of the fourth user input 170, the monitor device starts collection of image data received from the image sensor and temporarily stores the data in memory.

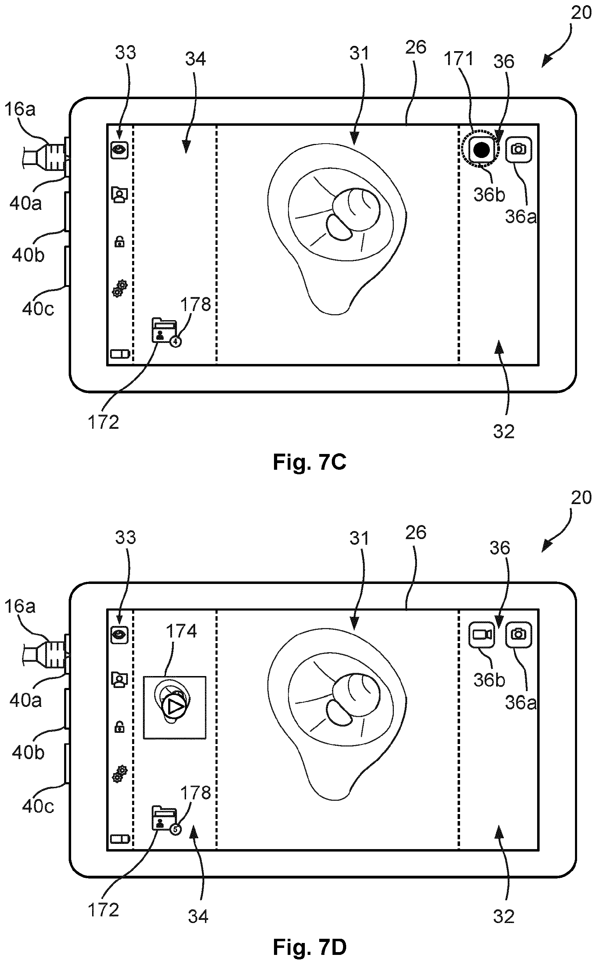

After detection of the fourth user input 170, the monitor device is adapted to detect a fifth user input 171 (as provided in Fig. 7C) corresponding to selection of the video capture button 36b.

In response to detection of the fifth user input 171, the monitor device stops the recording. More specifically, the monitor device stores a first video data file corresponding to the image data received between detection of the fourth user input 170 (Fig. 7A) and detection of the fifth user input 171. The monitor device 20, e.g. the processing unit of the monitor device 20, may read the temporarily stored data from the memory and create the first video data file based thereon. The monitor device 20 further associates the first video data file with the procedure session. For example, the monitor device 20 may store the first video data file in the folder corresponding to the procedure session.

Furthermore, as illustrated in Fig. 7D, the monitor device, in response to detection of the fifth user input 171 and storing of the first video data file displays within the background portion of the graphical user interface, e.g. the fourth portion 34 as illustrated, a fourth representation 174 corresponding to a frame of the stored first video data file. As illustrated, the representation 174 may comprise an indicator indicating that the representation corresponds to a video data file. In the present example, illustrated as a "play" icon. Similarly, an indicator may be provided on representations of an image file, which may differ from the indicator provided on representations of a video data file. Thus, an indicator may be provided to visually distinguish whether a video or a still image has been captured.

Furthermore, as also illustrated in Fig. 7D, the monitor device, in response to detection of the fifth user input 171 changes the appearance of the video capture button 36b to the first appearance.

Furthermore, also in response to detection of the fifth user input 170 and storing of the first video data file, the display of the visual representation 178 of the count of stored files stored during the procedure session is updated by increasing the count of stored files.

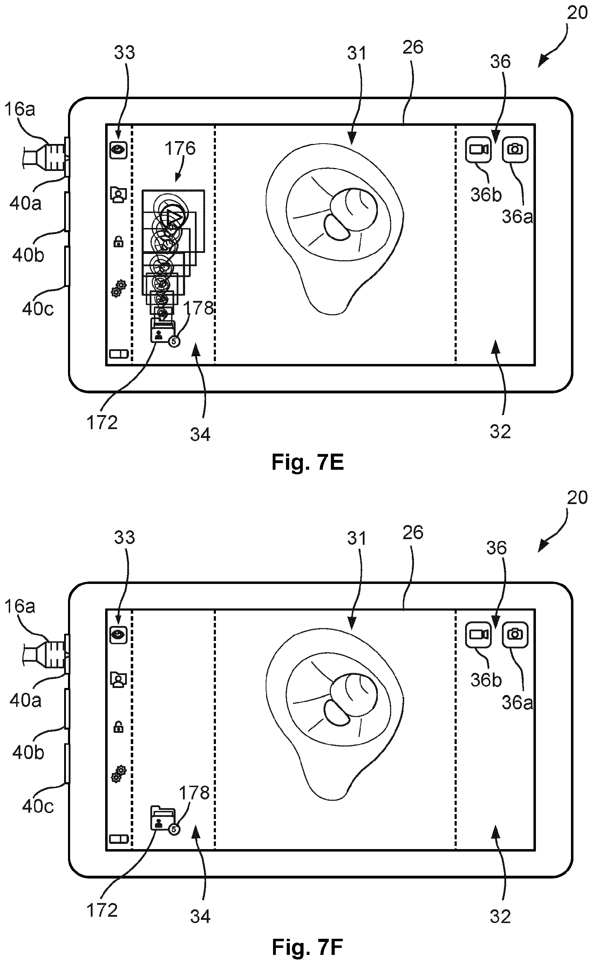

As shown in Fig. 7E, after a predetermined delay after detection of the fifth user input 171, the monitor device displays an animation 176 of transitioning the fourth representation 174 to the folder icon 172. The predetermined delay may be the same delay as explained with respect to Fig. 5C. The animation 166 may have a duration like the duration of the animation 156 as explained with respect to Fig. 5C.

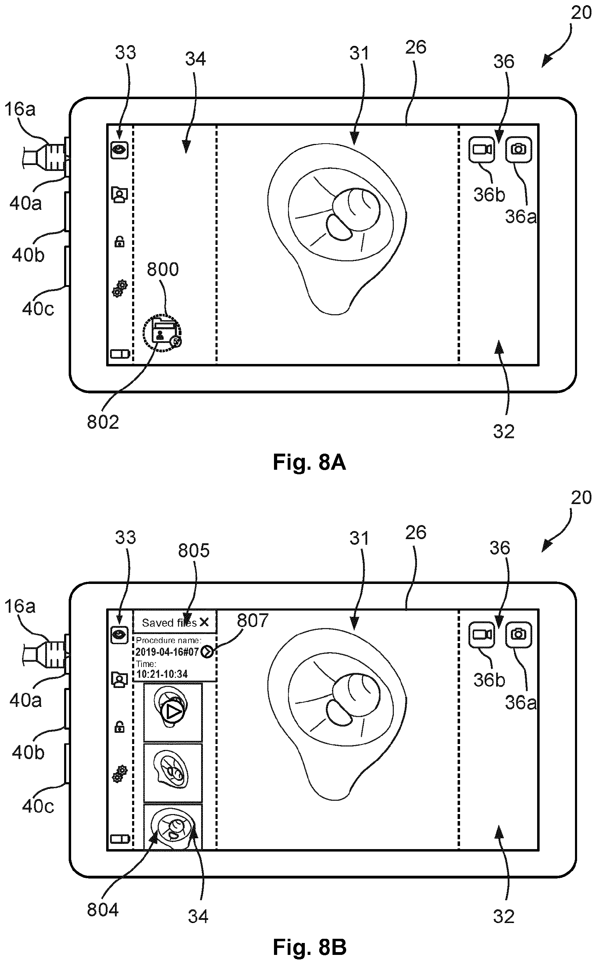

Figs. 8A-8H schematically illustrates exemplary user interactions with a graphical user interface of a monitor device 20, such as the monitor device 20 of any of the previous figures. The exemplary graphical user interface of Fig 8A may follow, e.g., after the exemplary graphical user interface of Fig. 7F. Thus, the folder icon 802 illustrated in Figs. 8A-8F may correspond to the folder icon 172 of Figs. 7A-7F, and the procedure session as referred to with respect to Figs. 8A-8F may be the same procedure session as explained in relation to Figs. 7A-7F.

As illustrated in Fig. 8A, a user may provide a sixth user input 800 corresponding to selection of the folder icon 802.The monitor device 20 is adapted to detect the sixth user input 800, and in response to detection of the sixth user input 800, as illustrated in Fig. 8B, the monitor device 20 displays, within the background portion (e.g. second portion or fourth portion 34) of the graphical user interface, a first plurality of representations 804 corresponding to a first plurality of stored image files stored during the procedure session. In the present example, first plurality of representations 804 is displayed within the fourth portion 34 of the graphical user interface. Alternatively, the first plurality of representations 804 may be displayed in the second portion 32 of the graphical user interface. Advantageously, the first plurality of representations 804 is displayed in the same portion as the folder icon 802.

Also, in response to detection of the sixth user input 800, a session info box 805 and a session overview icon 807 is displayed within the background portion of the graphical user interface, such as within the fourth portion 34 of the graphical user interface.

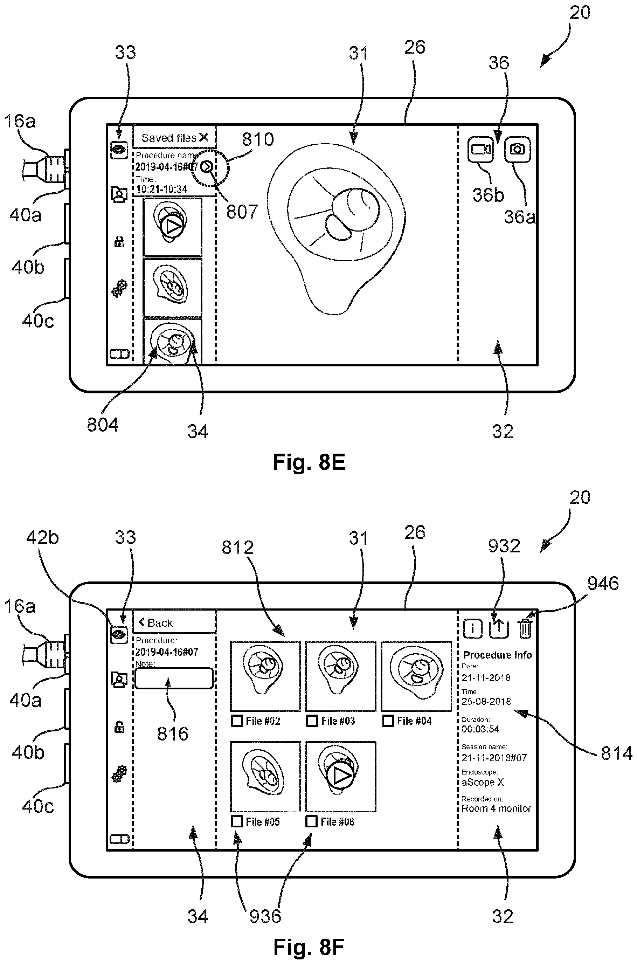

The exemplary graphical user interface of Fig. 8B may alternatively be reached by disconnecting the visualisation device from the monitor device 20. For example, in response to disconnection of the visualisation device from the monitor device 20, the monitor device 20 may display the graphical user interface as illustrated in Fig. 8B, e.g. comprising the first plurality of representations 804 corresponding to a first plurality of stored image files stored during the procedure session, and the session info box 805 and the session overview icon 807.

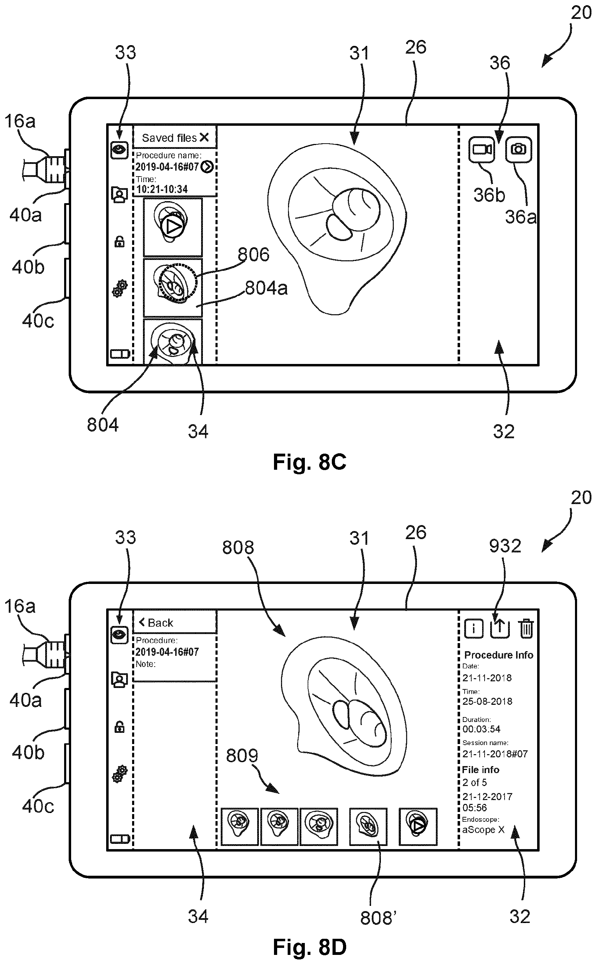

As illustrated in Fig. 8C, a user may provide a seventh user input 806 corresponding to selection of a primary representation 804a of the first plurality of representations 804. The primary representation 804a corresponds to a primary stored image file. The monitor device 20 is adapted to detect the seventh user input 806, and in response to detection of the seventh user input 806, as illustrated in Fig. 8D, the monitor device 20 displays an enlarged representation 808 of the primary stored image file within the first portion 31 of the graphical user interface. Furthermore, the monitor device 20 also displays thumbnail representations 809 of a second plurality of the stored image files stored during the procedure session. Displaying the thumbnail representations 809 differentiates the view from a live view, e.g. Fig. 3, thereby notifying the user that monitor device 20 is not currently showing a live representation of image data received from the visualisation device, but instead the monitor device 20 is displaying stored images.

As illustrated in Fig. 8E, originating from the situation as described with respect to Fig. 8B, a user may provide an eighth user input 810 corresponding to selection of the session overview icon 807. The monitor device 20 is adapted to detect the eighth user input 810, and in response to detection of the eighth user input 810, as illustrated in Fig. 8F, the monitor device 20 displays in the first portion of the graphical user interface a third plurality of representations 812 corresponding to a third plurality of stored image files stored during the procedure session. Furthermore, also in response to detection of the eighth user input 810, the monitor device displays general information 814 of the procedure session, e.g. in the second portion 32 of the graphical user interface, and a note field 816 in the fourth portion 34 of the graphical user interface.

The monitor device also displays an export icon 932 and a deletion icon 946, in the second portion of the graphical user interface. These will be described in more detail later.

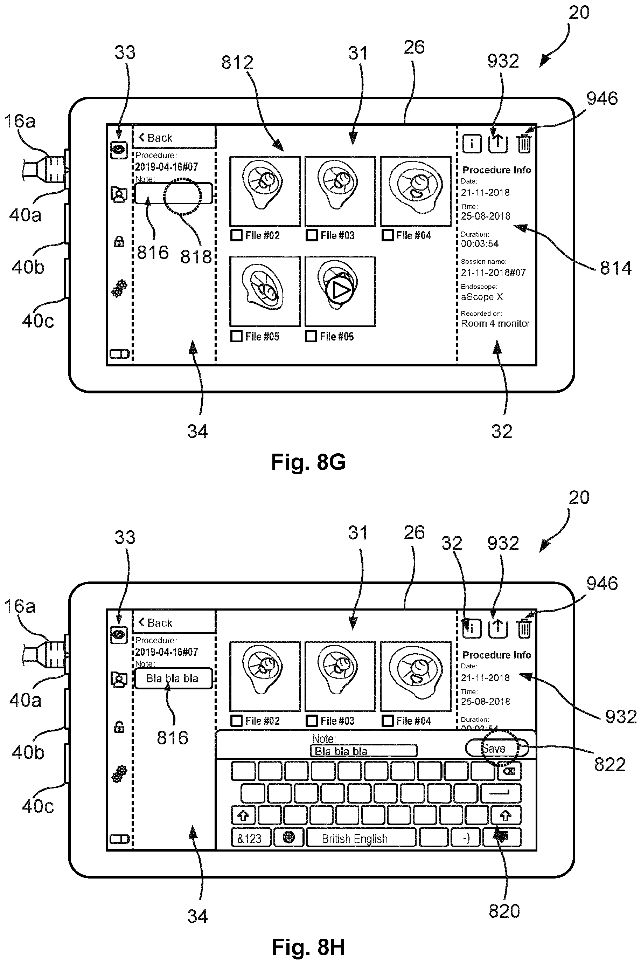

As illustrated in Fig. 8G a user may provide a ninth user input 818 corresponding to selection of the note field 816. The monitor device 20 is adapted to detect the ninth user input 818, and in response to detection of the ninth user input 818, as illustrated in Fig. 8H the monitor device 20 displays a virtual keyboard 820 in the first portion 31 of the graphical user interface and optionally extending into the second portion 32 of the graphical user interface. The virtual keyboard is configured for entering text in the note field 816. Thus, the monitor device 20 is further adapted to, e.g. with the touch sensitive display 26, detect a sequence of keyboard user inputs corresponding to typing of a text using the displayed virtual keyboard, and detect a tenth user input 822 indicative of acceptance of the text typed using the displayed virtual keyboard 820. For example, the tenth user input 822 may be corresponding to selection of a "save" button, as illustrated. In response to detection of the tenth user input 822, the monitor device 20 stores and associates the typed text as a note for the procedure session.

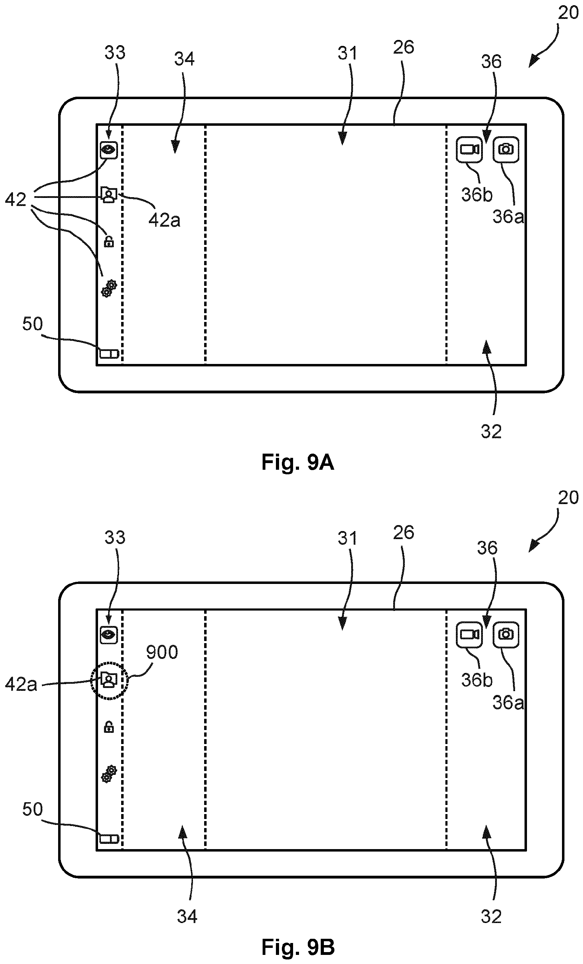

Figs. 9A-9L schematically illustrates exemplary user interactions with a graphical user interface of a monitor device 20, such as the monitor device 20 of any of the previous figures. The monitor device 20 may have a visualisation device connected, or it may not have a visualisation device connected.

With reference to Fig. 9A, the one or more actionable menu items 42 displayed in the third portion 33 of the graphical user interface comprises a first actionable menu item being an archive menu item 42a.

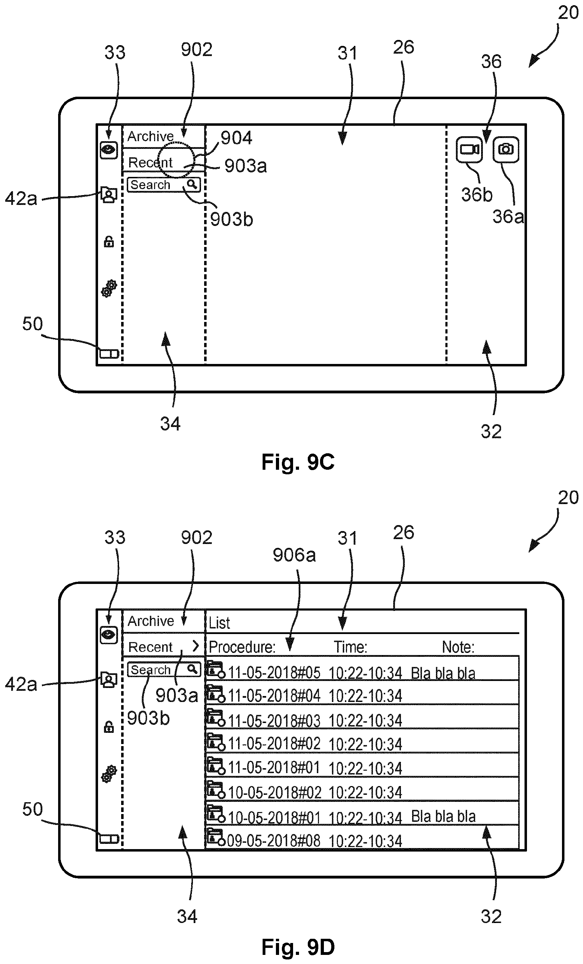

As illustrated in Fig. 9B, an eleventh user input 900 corresponding to selection of the archive menu item 42a may be received. The monitor device 20 is adapted to detect the eleventh user input 900, and in response to detection of the eleventh user input 900, as illustrated in Fig. 9C, the monitor device 20 displays a primary archive menu 902 associated with the archive menu item 42a within the fourth portion of the graphical user interface. The primary archive menu 902 comprises one or more primary actionable archive items including a first primary actionable archive item 903a. In the illustrated example, the one or more primary actionable archive items includes a first primary actionable item 903a to retrieve recent (e.g. recently stored) images and videos, and a second primary actionable item 903b to search for stored images and videos.

As illustrated in Fig. 9C, while displaying the primary archive menu 902, a twelfth user input 904 corresponding to selection of the first primary actionable archive item 903a may be received. The monitor device is adapted to detect the twelfth user input 904, and in response to detection of the twelfth user input 904, as illustrated in Fig. 9D, the monitor device 20 displays in the first portion of the graphical user interface, optionally extending into the second portion 32 and/or the fourth portion 34 of the graphical user interface, a secondary archive menu 906a associated with the first primary actionable archive item.

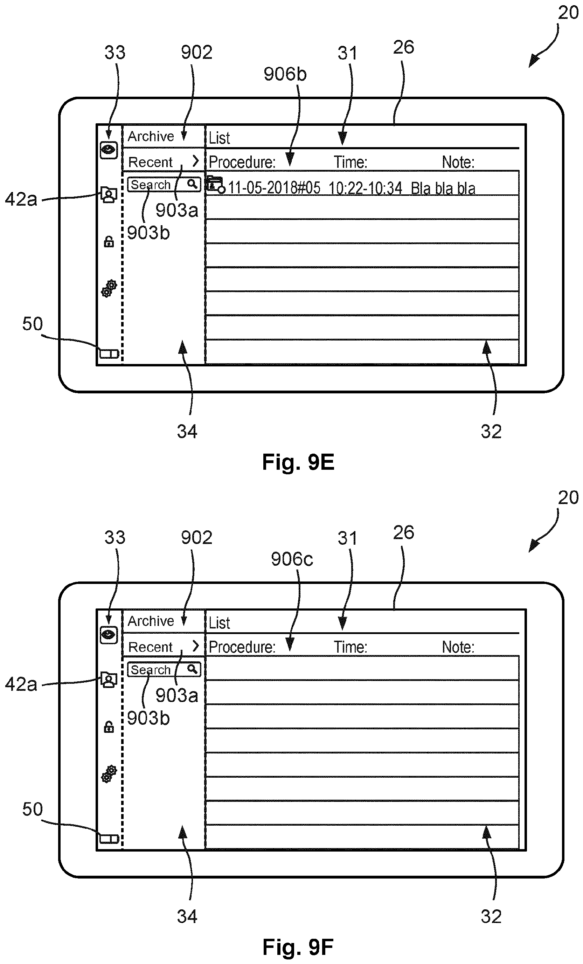

The content of the secondary archive menu may be subject to authorisation. For example, subject to whether a user is logged (e.g. having authenticated by typing in username and password) into the monitor device and whether the user being logged in has certain privileges. For example, in accordance with the monitor device operating in an authorised state, the secondary archive menu may comprise a list of stored, such as all stored, procedure sessions, e.g. such as the secondary archive menu 906a as illustrated in Fig. 9D. In accordance with the monitor device 20 operating in a non-authorised state and a setting to require authorisation is activated, the secondary archive menu may comprise an empty list, e.g. such as the secondary archive menu 906c as illustrated in Fig. 9F, or a list of a subset, e.g. such as the secondary archive menu 906b as illustrated in Fig. 9E, of the stored procedure sessions. In accordance with the monitor device 20 operating in a non-authorised state and a setting to require authorisation is deactivated, the secondary archive menu may comprise the list of stored procedure sessions, e.g. such as the secondary archive menu 906a as illustrated in Fig. 9D.

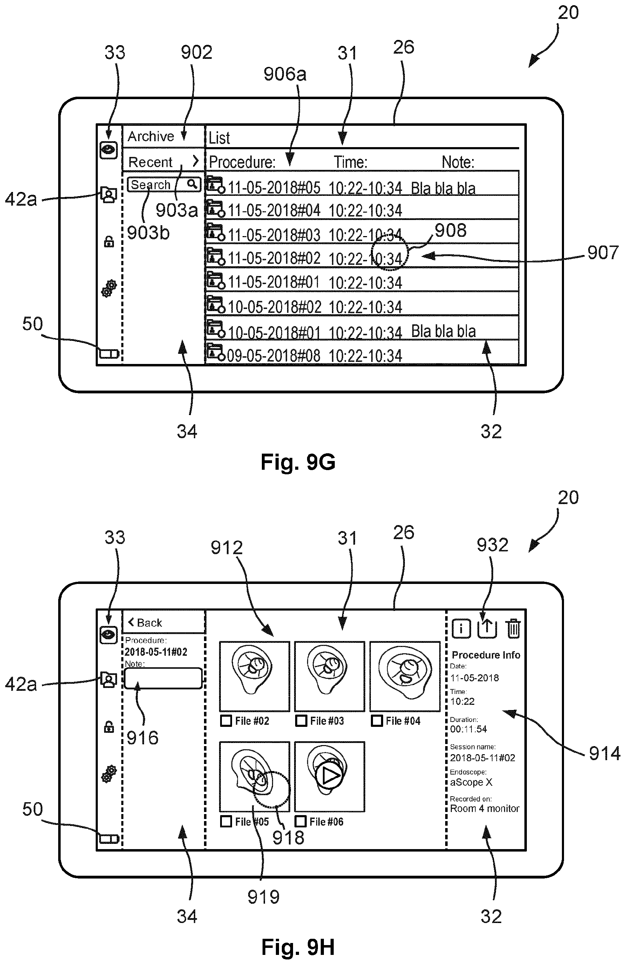

As illustrated in Fig. 9G, while displaying the secondary archive menu 906a (the illustrated example shows the secondary archive menu 906a in accordance with Fig. 9D. However, the same would apply for a secondary archive menu 906b in accordance with Fig. 9E), a thirteenth user input 908 corresponding to selection of a first stored procedure session 907. The monitor device is adapted to detect the thirteenth user input 908, and in response to detection of the thirteenth user input 908, as illustrated in Fig. 9H the monitor device displays in the first portion 31 of the graphical user interface a fourth plurality of representations 912 corresponding to a fourth plurality of stored image files stored during the first stored procedure session 907. Furthermore, also in response to detection of the thirteenth user input 908, the monitor device 20 displays general information 914 of the first stored procedure session 907, e.g. in the second portion 32 of the graphical user interface. Furthermore, also in response to detection of the thirteenth user input 908, the monitor device 20 displays a note field 916, e.g. in the fourth portion 34 of the graphical user interface.

In the event that the first stored procedure session 907 selected by the thirteenth user input 908 was the same as the procedure session of Figs. 8A-8H, the graphical user interface as provided in response to detection of the thirteenth user input 908, and as illustrated in Fig. 9H, would be the same graphical user interface as provided in response to detection of the eighth user input 810, and as illustrated in Fig. 8F. Thus, the disclosure with respect to selection of the notes field 816 by the ninth user input 818 as described with respect to Fig. 8F applies, mutatis mutandis to the situation of Fig. 9H. Similarly, the following disclosure with respect to Fig. 9H applies, mutatis mutandis to the situation of Fig. 8F.

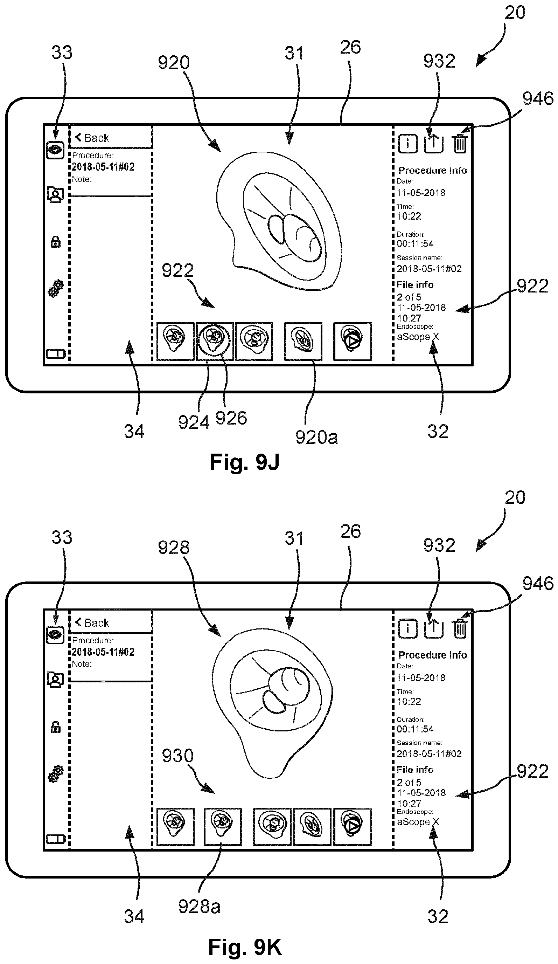

As illustrated in Fig. 9H, a fourteenth user input 918 corresponding to selection of a select representation 919 of the fourth plurality of representations 912 may be received. The select representation 919 corresponds to a select stored image file. The monitor device is adapted to detect the fourteenth user input 918, and in response to detection of the fourteenth user input 918, as illustrated in Fig. 9J, the monitor device displays an enlarged representation 920 of the select stored image file within the first portion 31 of the graphical user interface. Furthermore, also in response to the fourteenth user input 918, the monitor device 20, displays thumbnail representations 922 of a fifth plurality of the stored image files stored during the first stored procedure session 917. The thumbnail representations 922 comprises a thumbnail representation 920a of the select stored image file. The thumbnail representations 922 further comprises a further thumbnail 924 corresponding to a further stored image file.

Furthermore, also in response to detection of the fourteenth user input 918, the monitor device 20 displays, within the second portion 32 of the graphical user interface, image information 922 associated with the select stored image file. The image information 922 associated with the select stored image file comprise information indicative of the visualisation device. This information is saved based on the device identifier information obtained in response to detection of the visualisation device.

As illustrated in Fig. 9J, a fifteenth user input 926 corresponding to selection of a select thumbnail 924 of the displayed thumbnail representations 922 may be received. The select thumbnail corresponds to a second select stored image file. The monitor device is adapted to detect the fifteenth user input 926, and in response to detection of the fifteenth user input 926, as illustrated in Fig. 9K, the monitor device displays an enlarged representation 928 of the second select stored image file within the first portion 31 of the graphical user interface. The thumbnail representations 922 are updated to display representations 930 of a sixth plurality of the stored image files stored during the first stored procedure session. In the illustrated example, the thumbnail representations 922 of the fifth plurality of the stored image files, as seen in Fig. 9J, and the thumbnail representations 930 of the sixth plurality of the stored image files, as seen in Fig. 9K, are the same plurality of stored image files. However, in some examples, e.g. where the number of images are large, so that all images are unable to fit concurrently on the screen, the thumbnail representations may differ.

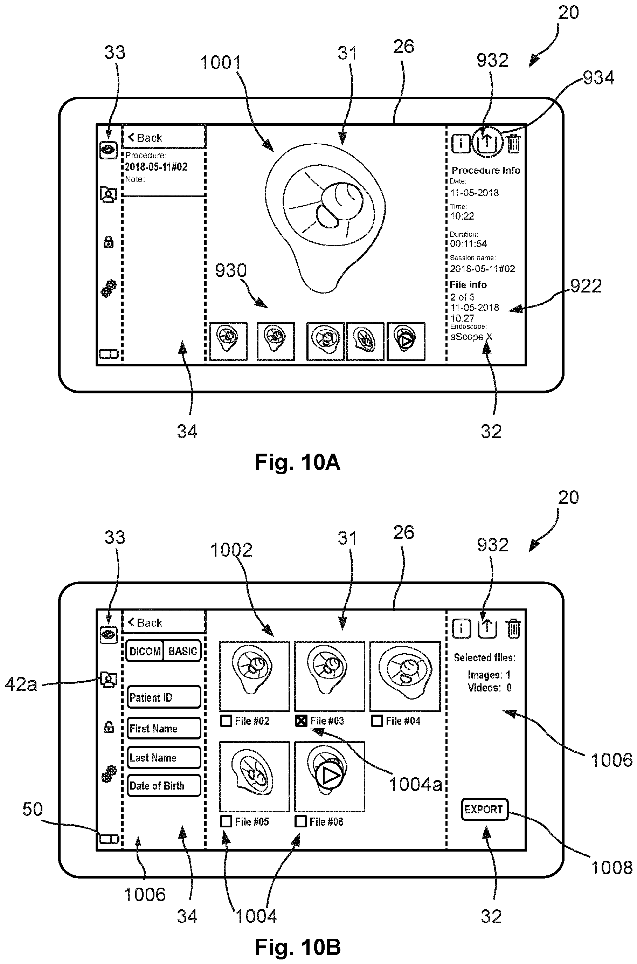

Figs. 10A-10D schematically illustrates exemplary user interactions with a graphical user interface of a monitor device 20, such as the monitor device 20 of any of the previous figures. The monitor device 20 may have a visualisation device connected, or it may not have a visualisation device connected. The exemplary graphical user interface of Fig 10A may follow, e.g., after the exemplary graphical user interfaces of Figs 9J, 9K or Fig. 8D. In the illustrated example, the monitor device 20 displays an enlarged representation 1001 of a stored image file. The enlarged representation 1001 may correspond to the enlarged representation 920 of the select stored image file of Fig. 9J, the enlarged representation 928 of the second select stored image file of Fig. 9K, or the enlarged representation 808 of the primary stored image file of Fig. 8D.

With reference to Fig. 10A, an export icon 932 is displayed, e.g. in the second portion of the graphical user interface. As illustrated, a sixteenth user input 934 corresponding to selection of the export icon 932 may be received. The monitor device 20 is adapted to detect the sixteenth user input 934, and in response to detection of the sixteenth user input 934, as illustrated in Fig. 10B, the monitor device displays in the first portion 31 of the graphical user interface a plurality of representations 1002 corresponding to a plurality of stored image files, e.g. the plurality of representations 1002 may be the fourth plurality of representations 912 corresponding to the fourth plurality of stored image files, as illustrated in relation to Fig. 9H. Each of the plurality of representations 1002 comprises a selection indicator 1004, wherein the selection indicator 1004a of the representation of the stored image file (of which the enlarged representation 1001 was displayed when the sixteenth user input 934 was detected) is activated.

Furthermore, also in response to detection of the sixteenth user input 934, the monitor device displays an export menu 1006 in the second portion 32 and fourth portion 34 of the graphical user interface. The export menu 1006 may be separated into two parts as illustrated. The export menu 1006 comprises an export confirm icon 1008.

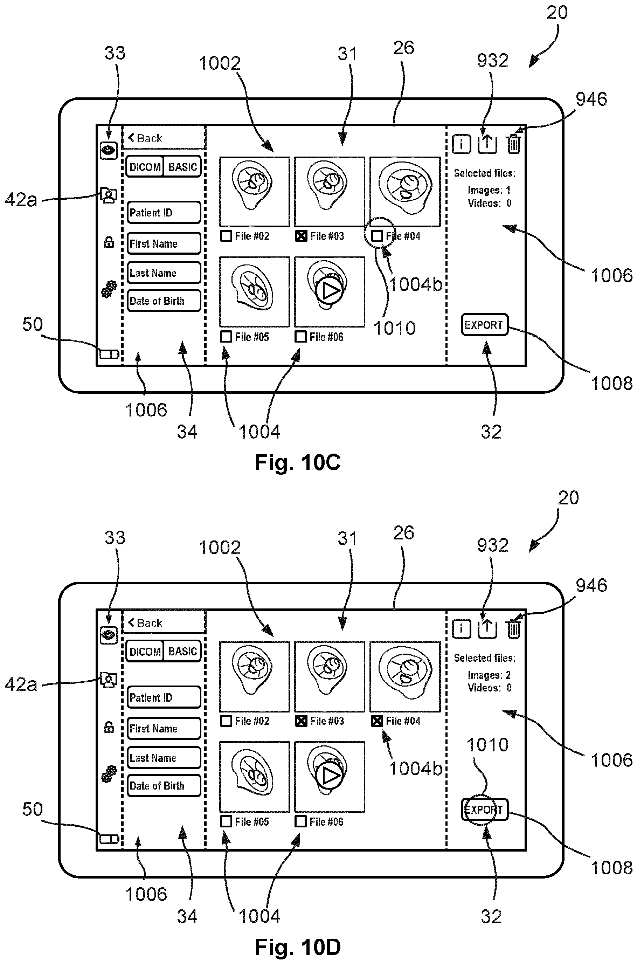

After detection of the sixteenth user input 934, and as illustrated in Fig. 10C, a seventeenth user input 1010, corresponding to selection of one or more of the selection indicators 1004 of the plurality of representations 1002, may be received. In the illustrated example, the seventeenth user input 1010 correspond to selection of a second selection indicator 1004b of the selection indicators 1004. The monitor device 20 is adapted to detect the seventeenth user input 1010, and in response to detection of the seventeenth user input 1010, the monitor device 20 activates the selection indicators corresponding to the selected one or more selection indicators. Hence, in the illustrated example, as illustrated in Fig. 10D, the second selection indicator 1004b is activated. Had the seventeenth user input 1010 corresponded to selection of an already selected selection indicator, e.g. the selection indicator 1004a, the monitor device 20 had deactivated the selection indicator 1004a.

As illustrated, the export menu 1006 comprises an export summary 1006 indicating the number of files and their type, which are about to be exported.

As illustrated the user may select type of export, e.g. DICOM or Basic (e.g. jpg, png, tiff etc.), and may input patient details, such as Patient ID, First Name, Last Name, Date of Birth, etc. In some examples, export via DICOM may be only available for users being authenticated.

As illustrated in Fig. 10D, after having selected the files for export, the user may provide an eighteenth user input 1010 corresponding to selection of the export confirm icon 1008. The monitor device is adapted to detect the eighteenth user input 1010, and in response to detection of the eighteenth user input 1010, the monitor device transmits to an auxiliary device, e.g. a USB drive or a server via a local or wide area network, stored image files corresponding to the selected one or more selection indicators 1004a, 1004b.

As seen, e.g. in Fig. 8F, following the eighth user input 810 corresponding to selection of the session overview icon 807 of Fig. 8E, and in Fig. 9H following the thirteenth user input 908 corresponding to selection of the first stored procedure session 907 of Fig. 9G, other graphical user interfaces may comprise the export icon 932. Providing an input corresponding to selection of the export icon 932 in these situations would result in the graphical user interface as exemplified with Fig. 10B and the associated description, with the modification that none of the selection indicators would be activated, i.e. none of the stored image files are pre-selected for export.

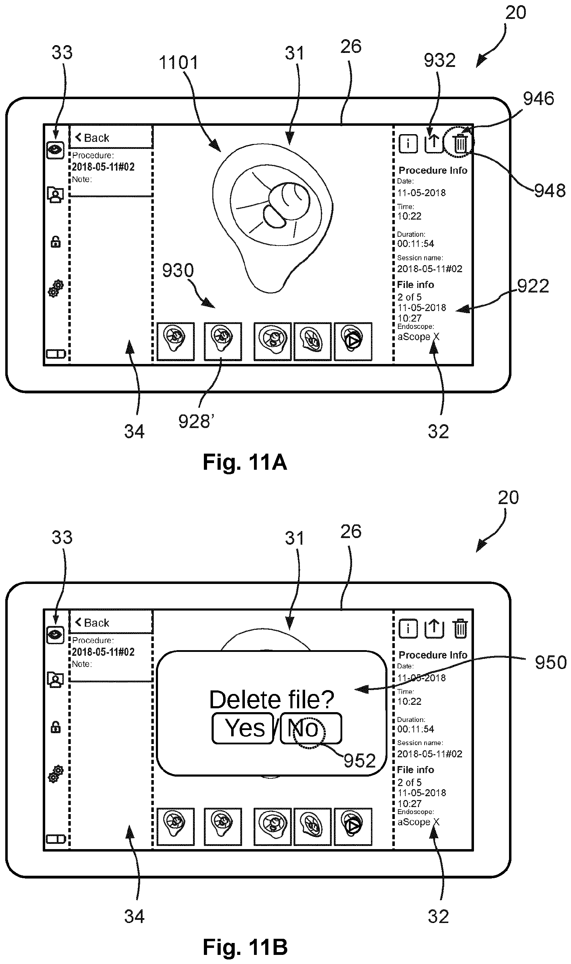

Figs. 11A-11E schematically illustrates exemplary user interactions with a graphical user interface of a monitor device 20, such as the monitor device 20 of any of the previous figures. The monitor device 20 may have a visualisation device connected, or it may not have a visualisation device connected. The exemplary graphical user interface of Fig 11A may follow, e.g., after the exemplary graphical user interfaces of Figs 9J, 9K or Fig. 8D. In the illustrated example, the monitor device 20 displays an enlarged representation 1101 of a stored image file. The enlarged representation 1101 may correspond to the enlarged representation 920 of the select stored image file of Fig. 9J, the enlarged representation 928 of the second select stored image file of Fig. 9K, or the enlarged representation 808 of the primary stored image file of Fig. 8D.

A nineteenth user input 948, corresponding to selection of the deletion icon 946, may be received. The monitor device 20 is adapted to detect the nineteenth user input 948, and in response to detection of the nineteenth user input 948, as illustrated in Fig. 11B, the monitor device 20 displays a confirmation dialogue 950 indicative of potential deletion of the stored image file (corresponding to the enlarged representation 1101 displayed when the nineteenth user input 948 was detected).

The user provides a twentieth user input 952 to the conformation dialogue 950. The monitor device is adapted to detect the twentieth user input 952 and determine whether the twentieth user input 952 is indicative of the user confirming deletion of the stored image file or of the user cancelling deletion of the select stored image file.

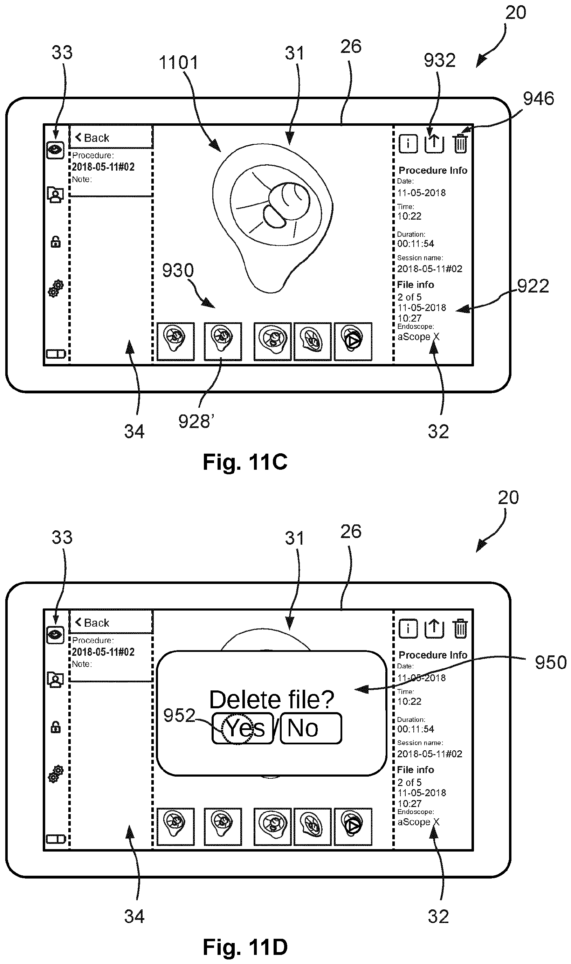

In one example, as illustrated in Fig. 11B, the twentieth user input 952 is indicative of the user cancelling deletion of the select stored image file. The monitor device accordingly foregoes deletion of the select stored image file and maintains display of the enlarged representation 1101 of the stored image file within the first portion 31 of the graphical user interface, as illustrated in Fig. 11C.

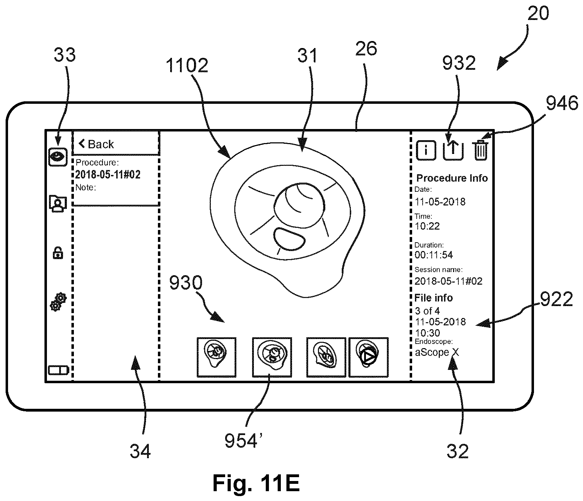

In another example, as illustrated in Fig. 11D, the twentieth user input 952 is indicative of the user confirming deletion of the stored image file. The monitor device accordingly deletes the stored image file and replaces the display of the enlarged representation 1101 of the stored image file with an enlarged representation 1102 of a second stored image file, as illustrated in Fig. 11E.

As seen, e.g. in Fig. 8F, following the eighth user input 810 corresponding to selection of the session overview icon 807 of Fig. 8E, and in Fig. 9H following the thirteenth user input 908 corresponding to selection of the first stored procedure session 907 of Fig. 9G, graphical user interfaces comprising the selection interface, as explained with respect to Figs. 10B-10D, may comprise the deletion icon 946. Multiple image files may be selected for deletion in accordance with the selection as explained with respect to Figs. 10B-10D, and a subsequent user input to the deletion icon 946 will displays a confirmation dialogue similar to the confirmation dialogue 950, indicative of potential deletion of the selected stored image files. A confirmation or cancellation similar to the description with respect to Figs. 11B and 11D will cause either deletion or cancellation of deletion of the selected stored image files.

Throughout the description, the use of the terms "first", "second", "third", "fourth", "primary", "secondary", "tertiary" etc. does not imply any particular order of importance but are included to identify individual elements. Furthermore, the labelling of a first element does not imply the presence of a second element and vice versa.

### LIST OF REFERENCES

- 2: medical visualisation system
- 4: visualisation device
- 6: handle
- 7: control button
- 8: elongated flexible member
- 9: distal part
- 10: distal end of elongated flexible member
- 12: image sensor
- 14: device cable
- 16: device connector
- 20: monitor device
- 21: first housing side
- 22: second housing side
- 23: third housing side
- 24: fourth housing side
- 25: first housing
- 26: touch sensitive display
- 27: graphical user interface
- 31: first portion
- 32: second portion
- 33: third portion
- 34: fourth portion
- 36: actionable item(s)
- 37: first image direction
- 38: second image direction
- 40: connection port(s)
- 42: actionable menu item(s)
- 44: invert view button
- 46: inverted view mode indicator
- 50: battery indicator
- 60: processing unit
- 61: power supply
- 61a: battery
- 61b: power connection
- 62: memory
- 64: orientation sensor
- 66: input/output
- 68: microphone
- 70: live representation of image data
- x1: first direction
- x2: second direction
- L1: first length
- L2: second length

## Claims

1. A medical visualisation system (2) comprising a visualisation device (4) having an image sensor (12) configured to generate image data indicative of a view from the visualisation device,
the medical visualisation system further comprising a monitor device (20) operable to receive the image data as the image data is being generated by the image sensor, the monitor device comprising a first housing extending in a first direction from a first housing side to a second housing side and in a second direction perpendicular to the first direction from a third housing side to a fourth housing side, the monitor device comprising a touch sensitive display accommodated in the first housing and having a first length in the first direction and a second length in the second direction, the monitor device displays with the touch sensitive display a graphical user interface (27),
wherein the monitor device (20):
- opens a procedure session;
- displays a live representation of the image data within a first portion of the graphical user interface;
- displays one or more actionable items within a second portion of the graphical user interface, wherein the one or more actionable items comprise an image capture button, the second portion and the first portion being non-overlapping; and
- displays a folder icon within a background portion of the graphical user interface, the background portion and the first portion being non-overlapping,
and wherein the monitor device is further adapted to detect a first user input corresponding to selection of the image capture button, and in response to detection of the first user input, the monitor device (20):
- stores a first image file corresponding to the image data received when the first user input was detected;
- associates the first image file with the procedure session; and
- displays within the background portion of the graphical user interface a first representation of a still image corresponding to the stored first image file;
after a predetermined delay after detection of the first user input, the monitor device (20) displays an animation of transitioning the first representation to the folder icon.

2. Medical visualisation system according to claim 1, wherein the monitor device is adapted to establish connection to the visualisation device including obtaining device identifier information from a device identifier of the visualisation device, and wherein the monitor device opens the procedure session in response to establishing the connection to the visualisation device, and the procedure session corresponds to the device identifier information.

3. Medical visualisation system according to claim 2, wherein the monitor device in opening the procedure session determines, based on the device identifier information, whether the visualisation device has been previously connected to the monitor device; and wherein
in accordance with determining that the visualisation device has previously been connected to the monitor device, the monitor device reopens the procedure session corresponding to the device identifier information; and
in accordance with determining that the visualisation device has not previously been connected to the monitor device, the monitor device creates the procedure session corresponding to the device identifier information.

4. Medical visualisation system according to any of the preceding claims, wherein the predetermined delay is between 1-5 seconds, such as between 1.5-3 seconds, such as 1.5 seconds or 2 seconds, and/or wherein the animation of transitioning the first representation to the folder icon has a duration between 100-1500 ms, such as between 300-1000 ms, such as between 300-600 ms, such as 400 ms or 500 ms.

5. Medical visualisation system according to any of the preceding claims, wherein the folder icon comprises a visual representation of a count of stored files stored during the procedure session, and wherein the monitor device in response to detection of the first user input, updates display of the visual representation of the count of stored files stored during the procedure session including increasing the count of stored files.

6. Medical visualisation system according to any of the preceding claims, wherein the monitor device, after detection of the first user input, is further adapted to detect a second user input corresponding to selection of the image capture button, and in response to detection of the second user input the monitor device:
- stores a second image file corresponding to the image data received when the second user input was detected;
- associates the second image file with the procedure session; and
- displays within the background portion of the graphical user interface a second representation of a still image corresponding to the stored second image file;
after the predetermined delay after detection of the second user input, the monitor device displays an animation of transitioning the second representation to the folder icon.

7. Medical visualisation system according to any of the preceding claims, wherein the monitor device is adapted to establish connection to a second visualisation device including obtaining second device identifier information from a second device identifier of the second visualisation device, in response to establishing the connection to the second visualisation device, the monitor device opens a second procedure session corresponding to the second device identifier information.

8. Medical visualisation system according to claim 7, wherein the monitor device:
- displays a live representation of second image data generated by a second image sensor of the second visualisation device;
- displays a second folder icon within the background portion of the graphical user interface;
and wherein the monitor device is adapted to detect a third user input corresponding to selection of the image capture button, and in response to detection of the third user input, the monitor device:
- stores a third image file corresponding to the second image data received when the third user input was detected; and
- associates the third image file with the second procedure session.

9. Medical visualisation system according to claim 8, wherein in response to detection of the third user input, the monitor device displays within the background portion of the graphical user interface a third representation of a still image corresponding to the stored third image file,
after the predetermined delay after detection of the third user input, the monitor device displays an animation of transitioning the third representation to the second folder icon.

10. Medical visualisation system according to any of the preceding claims, wherein the one or more actionable items comprises a video capture button displayed in a first appearance, and wherein the monitor device is adapted to detect a fourth user input corresponding to selection of the video capture button, and in response to detection of the fourth user input, the monitor device changes the appearance of the video capture button to a second appearance,
after detection of the fourth user input, the monitor device is adapted to detect a fifth user input corresponding to selection of the video capture button, and in response to detection of the fifth user input, the monitor device:
- changes the appearance of the video capture button to the first appearance;
- stores a first video data file corresponding to the image data received between detection of the fourth user input and the fifth user input;
- associates the first video data file with the procedure session; and
- displays within the background portion of the graphical user interface a fourth representation corresponding to a frame of the stored first video data file,
after the predetermined delay after detection of the fifth user input, the monitor device displays an animation transitioning the fourth representation to the folder icon.

11. Medical visualisation system according to any of the preceding claims wherein the monitor device is adapted to detect a sixth user input corresponding to selection of the folder icon, and in response to detection of the sixth user input, the monitor device displays, within the background portion of the graphical user interface, a first plurality of representations corresponding to a first plurality of stored image files stored during the procedure session.

12. Medical visualisation system according to claim 11 wherein the monitor device is further adapted to detect a seventh user input corresponding to selection of a primary representation of the first plurality of representations displayed in response to detection of the sixth user input, the primary representation corresponding to a primary stored image file, and in response to detection of the seventh user input the monitor device:
- displays an enlarged representation of the primary stored image file within the first portion of the graphical user interface; and
- displays thumbnail representations of a second plurality of the stored image files stored during the procedure session.

13. Medical visualisation system according to any of claims 11-12, wherein in response to detection of the sixth user input, the monitor device displays a session overview icon within the background portion of the graphical user interface,
the monitor device being adapted to detect an eighth user input corresponding to selection of the session overview icon, and in response to detection of the eighth user input the monitor device:
- displays in the first portion of the graphical user interface a third plurality of representations corresponding to a third plurality of stored image files stored during the procedure session;
- displays general information of the procedure session; and
- displays a note field.

14. Medical visualisation system according to claim 13 wherein the monitor device is further adapted to detect a ninth user input corresponding to selection of the note field, and in response to detection of the ninth user input the monitor device displays a virtual keyboard in the first portion of the graphical user interface and optionally extending into the second portion of the graphical user interface, the virtual keyboard being configured for entering text in the note field,
the monitor device being further adapted to:
- detect a sequence of keyboard user inputs corresponding to typing of a text using the displayed virtual keyboard; and
- detect a tenth user input indicative of accept of the text typed using the displayed virtual keyboard,
in response to detection of the tenth user input, the monitor device stores and associates the typed text as a note for the procedure session.

15. Medical visualisation system according to any of the preceding claims wherein in response to disconnection of the visualisation device from the monitor device, the monitor device displays, within the background portion of the graphical user interface, a first plurality of representations corresponding to a first plurality of stored image files stored during the procedure session, wherein optionally in response to disconnection of the visualisation device from the monitor device, the monitor device further displays a session overview icon within the background portion of the graphical user interface.

## Patentansprüche

1. Medizinisches Visualisierungssystem (2), umfassend eine Visualisierungsvorrichtung (4), die einen Bildsensor (12) aufweist, der konfiguriert ist, um Bilddaten zu erzeugen, die für eine Ansicht von der Visualisierungsvorrichtung bezeichnend sind,
wobei das medizinische Visualisierungssystem weiter eine Überwachungsvorrichtung (20) umfasst, die betreibbar ist, um die Bilddaten zu empfangen, während die Bilddaten durch den Bildsensor erzeugt werden, wobei die Überwachungsvorrichtung ein erstes Gehäuse umfasst, das sich in einer ersten Richtung von einer ersten Gehäuseseite zu einer zweiten Gehäuseseite und in einer zweiten Richtung senkrecht zu der ersten Richtung von einer dritten Gehäuseseite zu einer vierten Gehäuseseite erstreckt, wobei die Überwachungsvorrichtung eine berührungsempfindliche Anzeige umfasst, die in dem ersten Gehäuse untergebracht ist und eine erste Länge in der ersten Richtung und eine zweite Länge in der zweiten Richtung aufweist, die Überwachungsvorrichtung mit der berührungsempfindlichen Anzeige eine grafische Benutzeroberfläche (27) anzeigt,
wobei die Überwachungsvorrichtung (20):
- eine Prozedursitzung öffnet;
- eine Live-Darstellung der Bilddaten innerhalb eines ersten Abschnitts der grafischen Benutzeroberfläche anzeigt;
- ein oder mehrere betätigbare Elemente innerhalb eines zweiten Abschnitts der grafischen Benutzeroberfläche anzeigt, wobei das eine oder die mehreren betätigbaren Elemente eine Bildaufnahme-Schaltfläche umfassen, wobei der zweite Abschnitt und der erste Abschnitt nicht überlappend sind; und
- ein Ordnersymbol innerhalb eines Hintergrundabschnitts der grafischen Benutzeroberfläche anzeigt, wobei der Hintergrundabschnitt und der erste Abschnitt nicht überlappend sind,
und wobei die Überwachungsvorrichtung weiter angepasst ist, um eine erste Benutzereingabe, die der Auswahl der Bildaufnahme-Schaltfläche entspricht, zu erfassen, und die Überwachungsvorrichtung (20) als Reaktion auf das Erfassen der ersten Benutzereingabe:
- eine erste Bilddatei speichert, die den Bilddaten entspricht, die empfangen wurden, als die erste Benutzereingabe erfasst wurde;
- die erste Bilddatei mit der Prozedursitzung verknüpft; und
- innerhalb des Hintergrundabschnitts der grafischen Benutzeroberfläche eine erste Darstellung eines Standbilds, das der gespeicherten ersten Bilddatei entspricht, anzeigt;
wobei die Überwachungsvorrichtung (20) nach einer vorbestimmten Verzögerung nach dem Erfassen der ersten Benutzereingabe eine Animation eines Übergangs der ersten Darstellung zu dem Ordnersymbol anzeigt.

2. Medizinisches Visualisierungssystem nach Anspruch 1, wobei die Überwachungsvorrichtung angepasst ist, um eine Verbindung mit der Visualisierungsvorrichtung herzustellen, einschließlich eines Erhaltens von Vorrichtungskennungsinformationen von einer Vorrichtungskennung der Visualisierungsvorrichtung, und wobei die Überwachungsvorrichtung als Reaktion auf die Herstellung der Verbindung mit der Visualisierungsvorrichtung die Prozedursitzung öffnet, und die Prozedursitzung den Vorrichtungskennungsinformationen entspricht.

3. Medizinisches Visualisierungssystem nach Anspruch 2, wobei die Überwachungsvorrichtung beim Öffnen der Prozedursitzung basierend auf den Vorrichtungskennungsinformationen bestimmt, ob die Visualisierungsvorrichtung zuvor mit der Überwachungsvorrichtung verbunden wurde; und wobei
in Übereinstimmung mit dem Bestimmen, dass die Visualisierungsvorrichtung zuvor mit der Überwachungsvorrichtung verbunden wurde, die Überwachungsvorrichtung die Prozedursitzung, die den Vorrichtungskennungsinformationen entspricht, erneut öffnet; und
in Übereinstimmung mit dem Bestimmen, dass die Visualisierungsvorrichtung nicht zuvor mit der Überwachungsvorrichtung verbunden wurde, die Überwachungsvorrichtung die Prozedursitzung, die den Vorrichtungskennungsinformationen entspricht, erstellt.

4. Medizinisches Visualisierungssystem nach einem der vorstehenden Ansprüche, wobei die vorbestimmte Verzögerung zwischen 1-5 Sekunden, wie zwischen 1,5-3 Sekunden liegt, wie 1,5 Sekunden oder 2 Sekunden beträgt, und/oder wobei die Animation eines Übergangs der ersten Darstellung zu dem Ordnersymbol eine Dauer zwischen 100-1500 ms, wie zwischen 300-1000 ms, wie zwischen 300-600 ms, wie von 400 ms oder 500 ms aufweist.

5. Medizinisches Visualisierungssystem nach einem der vorstehenden Ansprüche, wobei das Ordnersymbol eine visuelle Darstellung einer Anzahl von gespeicherten Dateien, die während der Prozedursitzung gespeichert wurden, umfasst, und wobei die Überwachungsvorrichtung als Reaktion auf das Erfassen der ersten Benutzereingabe die Anzeige der visuellen Darstellung der Anzahl von gespeicherten Dateien, die während der Prozedursitzung gespeichert wurden, aktualisiert, einschließlich eines Erhöhens der Anzahl von gespeicherten Dateien.

6. Medizinisches Visualisierungssystem nach einem der vorstehenden Ansprüche, wobei die Überwachungsvorrichtung nach dem Erfassen der ersten Benutzereingabe weiter angepasst ist, um eine zweite Benutzereingabe, die der Auswahl der Bildaufnahme-Schaltfläche entspricht, zu erfassen, und die Überwachungsvorrichtung als Reaktion auf das Erfassen der zweiten Benutzereingabe:
- eine zweite Bilddatei speichert, die den Bilddaten entspricht, die empfangen wurden, als die zweite Benutzereingabe erfasst wurde;
- die zweite Bilddatei mit der Prozedursitzung verknüpft; und
- innerhalb des Hintergrundabschnitts der grafischen Benutzeroberfläche eine zweite Darstellung eines Standbilds, das der gespeicherten zweiten Bilddatei entspricht, anzeigt;
wobei die Überwachungsvorrichtung nach der vorbestimmten Verzögerung nach dem Erfassen der zweiten Benutzereingabe eine Animation eines Übergangs der zweiten Darstellung zu dem Ordnersymbol anzeigt.

7. Medizinisches Visualisierungssystem nach einem der vorstehenden Ansprüche, wobei die Überwachungsvorrichtung angepasst ist, um eine Verbindung mit einer zweiten Visualisierungsvorrichtung herzustellen, einschließlich eines Erhaltens zweiter Vorrichtungskennungsinformationen von einer zweiten Vorrichtungskennung der zweiten Visualisierungsvorrichtung, die Überwachungsvorrichtung als Reaktion auf die Herstellung der Verbindung mit der zweiten Visualisierungsvorrichtung eine zweite Prozedursitzung, die den zweiten Vorrichtungskennungsinformationen entspricht, öffnet.

8. Medizinisches Visualisierungssystem nach Anspruch 7, wobei die Überwachungsvorrichtung:
- eine Live-Darstellung von zweiten Bilddaten, die durch einen zweiten Bildsensor der zweiten Visualisierungsvorrichtung erzeugt werden, anzeigt;
- ein zweites Ordnersymbol innerhalb des Hintergrundabschnitts der grafischen Benutzeroberfläche anzeigt;
und wobei die Überwachungsvorrichtung angepasst ist, um eine dritte Benutzereingabe, die der Auswahl der Bildaufnahme-Schaltfläche entspricht, zu erfassen, und die Überwachungsvorrichtung als Reaktion auf das Erfassen der dritten Benutzereingabe:
- eine dritte Bilddatei speichert, die den zweiten Bilddaten entspricht, die empfangen wurden, als die dritte Benutzereingabe erfasst wurde; und
- die dritte Bilddatei mit der zweiten Prozedursitzung verknüpft.

9. Medizinisches Visualisierungssystem nach Anspruch 8, wobei die Überwachungsvorrichtung als Reaktion auf das Erfassen der dritten Benutzereingabe innerhalb des Hintergrundabschnitts der grafischen Benutzeroberfläche eine dritte Darstellung eines Standbilds, das der gespeicherten dritten Bilddatei entspricht, anzeigt,
wobei die Überwachungsvorrichtung nach der vorbestimmten Verzögerung nach dem Erfassen der dritten Benutzereingabe eine Animation eines Übergangs der dritten Darstellung zu dem zweiten Ordnersymbol anzeigt.

10. Medizinisches Visualisierungssystem nach einem der vorstehenden Ansprüche, wobei das eine oder die mehreren betätigbaren Elemente eine Videoaufnahme-Schaltfläche umfassen, die in einer ersten Erscheinung angezeigt ist, und wobei die Überwachungsvorrichtung angepasst ist, um eine vierte Benutzereingabe, die der Auswahl der Videoaufnahme-Schaltfläche entspricht, zu erfassen, und die Überwachungsvorrichtung als Reaktion auf das Erfassen der vierten Benutzereingabe, die Erscheinung der Videoaufnahme-Schaltfläche zu einer zweiten Erscheinung ändert,
wobei die Überwachungsvorrichtung nach dem Erfassen der vierten Benutzereingabe angepasst ist, um eine fünfte Benutzereingabe, die der Auswahl der Videoaufnahme-Schaltfläche entspricht, zu erfassen, und die Überwachungsvorrichtung als Reaktion auf das Erfassen der fünften Benutzereingabe:
- die Erscheinung der Videoaufnahme-Schaltfläche zu der ersten Erscheinung ändert;
- eine erste Videodatendatei speichert, die den Bilddaten entspricht, die zwischen dem Erfassen der vierten Benutzereingabe und der fünften Benutzereingabe empfangen wurden;
- die erste Videodatendatei mit der Prozedursitzung verknüpft; und
- innerhalb des Hintergrundabschnitts der grafischen Benutzeroberfläche eine vierte Darstellung, die einem Rahmen der gespeicherten ersten Videodatendatei entspricht, anzeigt,
wobei die Überwachungsvorrichtung nach der vorbestimmten Verzögerung nach dem Erfassen der fünften Benutzereingabe eine Animation eines Übergangs der vierten Darstellung zu dem Ordnersymbol anzeigt.

11. Medizinisches Visualisierungssystem nach einem der vorstehenden Ansprüche wobei die Überwachungsvorrichtung angepasst ist, um eine sechste Benutzereingabe, die der Auswahl des Ordnersymbols entspricht, zu erfassen, und die Überwachungsvorrichtung als Reaktion auf das Erfassen der sechsten Benutzereingabe innerhalb des Hintergrundabschnitts der grafischen Benutzeroberfläche eine erste Vielzahl von Darstellungen, die einer ersten Vielzahl von gespeicherten Bilddateien entspricht, die während der Prozedursitzung gespeichert wurden, anzeigt.

12. Medizinisches Visualisierungssystem nach Anspruch 11, wobei die Überwachungsvorrichtung weiter angepasst ist, um eine siebte Benutzereingabe zu erfassen, die der Auswahl einer primären Darstellung der ersten Vielzahl von Darstellungen entspricht, die als Reaktion auf das Erfassen der sechsten Benutzereingabe angezeigt werden, wobei die primäre Darstellung einer primären gespeicherten Bilddatei entspricht, und die Überwachungsvorrichtung als Reaktion auf das Erfassen der siebten Benutzereingabe:
- eine vergrößerte Darstellung der primären gespeicherten Bilddatei innerhalb eines ersten Abschnitts der grafischen Benutzeroberfläche anzeigt; und
- Miniaturdarstellungen einer zweiten Vielzahl der gespeicherten Bilddateien, die während der Prozedursitzung gespeichert wurden, anzeigt.

13. Medizinisches Visualisierungssystem nach einem der Ansprüche 11-12, wobei die Überwachungsvorrichtung als Reaktion auf das Erfassen der sechsten Benutzereingabe innerhalb des Hintergrundabschnitts der grafischen Benutzeroberfläche ein Sitzungsübersichtssymbol anzeigt,
wobei die Überwachungsvorrichtung angepasst ist, um eine achte Benutzereingabe, die der Auswahl des Sitzungsübersichtssymbols entspricht, zu erfassen, und die Überwachungsvorrichtung als Reaktion auf das Erfassen der achten Benutzereingabe:
- in dem ersten Abschnitt der grafischen Benutzeroberfläche eine dritte Vielzahl von Darstellungen anzeigt, die einer dritten Vielzahl von gespeicherten Bilddateien entspricht, die während der Prozedursitzung gespeichert wurden, anzeigt;
- allgemeine Informationen der Prozedursitzung anzeigt; und
- ein Notizfeld anzeigt.

14. Medizinisches Visualisierungssystem nach Anspruch 13, wobei die Überwachungsvorrichtung weiter angepasst ist, um eine neunte Benutzereingabe, die der Auswahl des Notizfelds entspricht, zu erfassen, und die Überwachungsvorrichtung als Reaktion auf das Erfassen der neunten Benutzereingabe eine virtuelle Tastatur in dem ersten Abschnitt der grafischen Benutzeroberfläche anzeigt, die sich optional in den zweiten Abschnitt der grafischen Benutzeroberfläche erstreckt,
wobei die virtuelle Tastatur zum Eingeben von Text in das Notizfeld konfiguriert ist,
wobei die Überwachungsvorrichtung weiter angepasst ist, um:
- eine Folge von Tastaturbenutzereingaben zu erfassen, die dem Eintippen eines Textes unter Verwendung der angezeigten virtuellen Tastatur entsprechen; und
- eine zehnte Benutzereingabe zu erfassen, die für das Akzeptieren des unter Verwendung der angezeigten virtuellen Tastatur eingetippten Textes bezeichnend ist,
wobei die Überwachungsvorrichtung als Reaktion auf das Erfassen der zehnten Benutzereingabe den eingetippten Text als eine Notiz für die Prozedursitzung speichert und verknüpft.

15. Medizinisches Visualisierungssystem nach einem der vorstehenden Ansprüche wobei die Überwachungsvorrichtung als Reaktion auf eine Trennung der Visualisierungsvorrichtung von der Überwachungsvorrichtung innerhalb des Hintergrundabschnitts der grafischen Benutzeroberfläche eine erste Vielzahl von Darstellungen, die einer ersten Vielzahl von gespeicherten Bilddateien entspricht, die während der Prozedursitzung gespeichert wurden, anzeigt, wobei optional die Überwachungsvorrichtung als Reaktion auf die Trennung der Visualisierungsvorrichtung von der Überwachungsvorrichtung innerhalb des Hintergrundabschnitts der grafischen Benutzeroberfläche weiter ein Sitzungsübersichtssymbol anzeigt.

## Revendications

1. Système de visualisation médicale (2) comprenant un dispositif de visualisation (4) présentant un capteur (12) d'image configuré pour générer des données d'image indicatives d'une vue provenant du dispositif de visualisation,
le système de visualisation médicale comprenant en outre un dispositif moniteur (20) servant à recevoir les données d'image quand les données d'image sont générées par le capteur d'image, le dispositif moniteur comprenant un premier boîtier s'étendant dans une première direction depuis un premier côté de boîtier vers un deuxième côté de boîtier et dans une deuxième direction perpendiculaire à la première direction depuis un troisième côté de boîtier vers un quatrième côté de boîtier, le dispositif moniteur comprenant un écran tactile logé dans le premier boîtier et présentant une première longueur dans la première direction et une deuxième longueur dans la deuxième direction, le dispositif moniteur affichant, à l'aide de l'écran tactile, une interface utilisateur graphique (27),
dans lequel le dispositif moniteur (20) :
- ouvre une session de procédure ;
- affiche une représentation en direct des données d'image dans une première partie de l'interface utilisateur graphique ;
- affiche un ou plusieurs éléments actionnables dans une deuxième partie de l'interface utilisateur graphique, dans lequel les un ou plusieurs éléments actionnables comprennent un bouton de capture d'image, la deuxième partie et la première partie ne se chevauchant pas ; et
- affiche une icône dossier dans une partie d'arrière-plan de l'interface utilisateur graphique, la partie d'arrière-plan et la première partie ne se chevauchant pas,
et dans lequel le dispositif moniteur est en outre adapté pour détecter une première entrée utilisateur correspondant à une sélection du bouton de capture d'image, et en réponse à la détection de la première entrée utilisateur, le dispositif moniteur (20) :
- stocke un premier fichier image correspondant aux données d'image reçues lorsque la première entrée utilisateur a été détectée ;
- associe le premier fichier image à la session de procédure ; et
- affiche dans la partie d'arrière-plan de l'interface utilisateur graphique une première représentation d'une image fixe correspondant au premier fichier image stocké ;
après un délai prédéterminé après la détection de la première entrée utilisateur, le dispositif moniteur (20) affiche une animation de transition de la première représentation vers l'icône dossier.

2. Système de visualisation médicale selon la revendication 1, dans lequel le dispositif moniteur est adapté à établir une connexion au dispositif de visualisation incluant une obtention d'informations d'identifiant de dispositif à partir d'un identifiant de dispositif du dispositif de visualisation, et dans lequel le dispositif moniteur ouvre la session de procédure en réponse à l'établissement de la connexion au dispositif de visualisation, et la session de procédure correspond aux informations d'identifiant de dispositif.

3. Système de visualisation médicale selon la revendication 2, dans lequel le dispositif moniteur lors de l'ouverture de la session de procédure détermine, sur base des informations d'identifiant de dispositif, si le dispositif de visualisation a été préalablement connecté au dispositif moniteur ; et dans lequel
conformément à la détermination que le dispositif de visualisation a été préalablement connecté au dispositif moniteur, le dispositif moniteur réouvre la session de procédure correspondant aux informations d'identifiant de dispositif ; et
conformément à la détermination que le dispositif de visualisation n'a pas été préalablement connecté au dispositif moniteur, le dispositif moniteur crée la session de procédure correspondant aux informations d'identifiant de dispositif.

4. Système de visualisation médicale selon l'une quelconque des revendications précédentes, dans lequel le délai prédéterminé est entre 1 et 5 secondes, tel qu'entre 1,5 et 3 secondes, tel que 1,5 secondes ou 2 secondes, et/ou dans lequel l'animation de transition de la première représentation vers l'icône dossier présente une durée entre 100 et 1500 ms, telle qu'entre 300 et 1000 ms, telle qu'entre 300 et 600 ms, telle que 400 ms ou 500 ms.

5. Système de visualisation médicale selon l'une quelconque des revendications précédentes, dans lequel l'icône dossier comprend une représentation visuelle d'un nombre de fichiers stockés qui sont stockés durant la session de procédure, et dans lequel le dispositif moniteur, en réponse à la détection de la première entrée utilisateur, met à jour l'affichage de la représentation visuelle du nombre de fichiers stockés durant la session de procédure incluant une augmentation du nombre de fichiers stockés.

6. Système de visualisation médicale selon l'une quelconque des revendications précédentes, dans lequel le dispositif moniteur, après détection de la première entrée utilisateur, est en outre adapté pour détecter une deuxième entrée utilisateur correspondant à la sélection du bouton de capture d'image, et en réponse à la détection de la deuxième entrée utilisateur, le dispositif moniteur :
- stocke un deuxième fichier image correspondant aux données d'image reçues lorsque la deuxième entrée utilisateur a été détectée ;
- associe le deuxième fichier image à la session de procédure ; et
- affiche dans la partie d'arrière-plan de l'interface utilisateur graphique une deuxième représentation d'une image fixe correspondant au deuxième fichier image stocké ;
après le délai prédéterminé après la détection de la deuxième entrée utilisateur, le dispositif moniteur affiche une animation de transition de la deuxième représentation vers l'icône dossier.

7. Système de visualisation médicale selon l'une quelconque des revendications précédentes, dans lequel le dispositif moniteur est adapté à établir une connexion à un deuxième dispositif de visualisation incluant une obtention d'informations d'identifiant de deuxième dispositif à partir d'un identifiant de deuxième dispositif du deuxième dispositif de visualisation, en réponse à l'établissement de la connexion au deuxième dispositif de visualisation, le dispositif moniteur ouvre une deuxième session de procédure correspondant aux informations d'identifiant de deuxième dispositif.

8. Système de visualisation médicale selon la revendication 7, dans lequel le dispositif moniteur :
- affiche une représentation en direct de deuxièmes données d'image générées par un deuxième capteur d'image du deuxième dispositif de visualisation ;
- affiche une deuxième icône dossier dans la partie d'arrière-plan de l'interface utilisateur graphique ;
et dans lequel le dispositif moniteur est adapté pour détecter une troisième entrée utilisateur correspondant à la sélection du bouton de capture d'image, et en réponse à la détection de la troisième entrée utilisateur, le dispositif moniteur :
- stocke un troisième fichier image correspondant aux deuxièmes données d'image reçues lorsque la troisième entrée utilisateur a été détectée ; et
- associe le troisième fichier image à la deuxième session de procédure.

9. Système de visualisation médicale selon la revendication 8, dans lequel en réponse à la détection de la troisième entrée utilisateur, le dispositif moniteur affiche dans la partie d'arrière-plan de l'interface utilisateur graphique une troisième représentation d'une image fixe correspondant au troisième fichier image stocké,
après le délai prédéterminé après la détection de la troisième entrée utilisateur, le dispositif moniteur affiche une animation de transition de la troisième représentation vers la deuxième icône dossier.

10. Système de visualisation médicale selon l'une quelconque des revendications précédentes, dans lequel les un ou plusieurs éléments actionnables comprennent un bouton de capture de vidéo affiché dans un premier aspect, et dans lequel le dispositif moniteur est adapté pour détecter une quatrième entrée utilisateur correspondant à la sélection du bouton de capture d'image, et en réponse à la détection de la quatrième entrée utilisateur, le dispositif moniteur change l'aspect du bouton de capture de vidéo en un deuxième aspect, après détection de la quatrième entrée utilisateur, le dispositif moniteur est adapté pour détecter une cinquième entrée utilisateur correspondant à la sélection du bouton de capture de vidéo, et en réponse à la détection de la cinquième entrée utilisateur, le dispositif moniteur :
- change l'aspect du bouton de capture de vidéo dans le premier aspect ;
- stocke un premier fichier de données vidéo correspondant aux données d'image reçues entre la détection de la quatrième entrée utilisateur et la cinquième entrée utilisateur ;
- associe le premier fichier de données vidéo à la session de procédure ; et
- affiche dans la partie d'arrière-plan de l'interface utilisateur graphique une quatrième représentation correspondant à une trame du premier fichier de données vidéo stocké,
après le délai prédéterminé après la détection de la cinquième entrée utilisateur, le dispositif moniteur affiche une animation de transition de la quatrième représentation vers l'icône dossier.

11. Système de visualisation médicale selon l'une quelconque des revendications précédentes dans lequel le dispositif moniteur est adapté pour détecter une sixième entrée utilisateur correspondant à la sélection de l'icône dossier, et en réponse à la détection de la sixième entrée utilisateur, le dispositif moniteur affiche, dans la partie d'arrière-plan de l'interface utilisateur graphique, une première pluralité de représentations correspondant à une première pluralité de fichiers d'image stockés qui sont stockés durant la session de procédure.

12. Système de visualisation médicale selon la revendication 11 dans lequel le dispositif moniteur est en outre adapté pour détecter une septième entrée utilisateur correspondant à la sélection d'une représentation primaire de la première pluralité de représentations affichées en réponse à la détection de la sixième entrée utilisateur, la représentation primaire correspondant à un fichier d'image stocké primaire, et en réponse à la détection de la septième entrée utilisateur le dispositif moniteur :
- affiche une représentation agrandie du fichier d'image stocké primaire dans la première partie de l'interface utilisateur graphique ; et
- affiche des représentations en vignette d'une deuxième pluralité des fichiers d'image stockés qui sont stockés durant la session de procédure.

13. Système de visualisation médicale selon l'une quelconque des revendications 11-12, dans lequel en réponse à la détection de la sixième entrée utilisateur, le dispositif moniteur affiche une icône aperçu de session dans la partie d'arrière-plan de l'interface utilisateur graphique,
le dispositif moniteur étant adapté pour détecter une huitième entrée utilisateur correspondant à la sélection de l'icône aperçu de session, et en réponse à la détection de la huitième entrée utilisateur, le dispositif moniteur :
- affiche dans la première partie de l'interface utilisateur graphique une troisième pluralité de représentations correspondant à une troisième pluralité de fichiers d'image stockés qui sont stockés durant la session de procédure ;
- affiche des informations générales de la session de procédure ; et
- affiche un champ note.

14. Système de visualisation médicale selon la revendication 13 dans lequel le dispositif moniteur est en outre adapté pour détecter une neuvième entrée utilisateur correspondant à une sélection du champ note, et en réponse à la détection de la neuvième entrée utilisateur le dispositif moniteur affiche un clavier virtuel dans la première partie de l'interface utilisateur graphique et s'étendant facultativement dans la deuxième partie de l'interface utilisateur graphique, le clavier virtuel étant configuré pour entrer du texte dans le champ note,
le dispositif moniteur étant en outre adapté pour :
- détecter une séquence d'entrées utilisateur au clavier correspondant à une saisie d'un texte en utilisant le clavier virtuel affiché ; et
- détecter une dixième entrée utilisateur indicative d'une acceptation du texte saisi en utilisant le clavier virtuel affiché,
en réponse à la détection de la dixième entrée utilisateur, le dispositif moniteur stocke et associe le texte saisi en tant que note pour la session de procédure.

15. Système de visualisation médicale selon l'une quelconque des revendications précédentes dans lequel en réponse à une déconnexion du dispositif de visualisation vis-à-vis du dispositif moniteur, le dispositif moniteur affiche, dans la partie d'arrière-plan de l'interface utilisateur graphique, une première pluralité de représentations correspondant à une première pluralité de fichiers d'image stockés qui sont stockés durant la session de procédure, dans lequel facultativement en réponse à la déconnexion du dispositif de visualisation vis-à-vis du dispositif moniteur, le dispositif moniteur affiche en outre une icône aperçu de session dans la partie d'arrière-plan de l'interface utilisateur graphique.
